# EUROPEAN PATENT APPLICATION

(11) **EP 4 578 490 A2**
(43) Date of publication of application: **02.07.2025**
(21) Application number: 25176189.6
(22) Date of filing: 15.12.2020
(51) Int. Cl.: A61N 1/378

(54) **STENT-ELECTRODE INTRAVASCULAR NEUROMODULATOR**

(30) Priority: 16.12.2019 US 201962948593 P
(62) Divisional of application: 20838159.0
(71) Applicant: Galvani Bioelectronics Limited, Stevenage SG1 2NY (GB)
(72) Inventor: BASHIRULLAH, Rizwan, South San Francisco, 94080 (US); HUNSBERGER, Gerald Edwin, Collegeville, 19426 (US)
(74) Representative: Carpmaels & Ransford LLP

(57) **Abstract**

A stent for intravascular stimulation comprises a scaffold comprising first and second scaffold structures, each scaffold structure comprising at least one substantially annular portion. The stent further comprises one or more anodal electrodes formed from or electrically coupled to at least a substantially annular portion of the first scaffold structure and one or more cathodal electrodes electrically formed from or coupled to at least a substantially annular portion of the second scaffold structure. The stent further comprises an anodal lead electrically coupled to the first scaffold structure to form a conductive path from the one or more anodal electrodes to a generator and a cathodal lead electrically coupled to the second scaffold structure to form a conductive path from the one or more cathodal electrodes to the generator. The stent further comprises a sleeve of insulating material, wherein the scaffold structures are attached to or formed on the sleeve of insulating material and are separated from each other by a distance such that the first and second scaffold structures are electrically insulated from each other.

## Description

### Field

This disclosure relates to neuromodulation devices and methods for non-destructively stimulating a nerve.

### Background

Electrical devices of various shapes and sizes including one or more electrode have been used for neurostimulation of target anatomy. An intravascular device which can effectively stimulate a target nerve is desirable, as such intravascular device may be less invasive than other forms of devices with electrodes.

### Summary

In one aspect, the invention provides a neuromodulation device (or a neurostimulation device) in the form of a stent for intravascular stimulation, the stent comprising: a scaffold comprising first and second scaffold structures, each scaffold structure comprising at least one substantially annular portion; one or more anodal electrodes formed from or electrically coupled to at least a substantially annular portion of the first scaffold structure and one or more cathodal electrodes electrically formed from or coupled to at least a substantially annular portion of the second scaffold structure; an anodal lead electrically coupled to the first scaffold structure to form a conductive path from the one or more anodal electrodes to a generator and a cathodal lead electrically coupled to the second scaffold structure to form a conductive path from the one or more cathodal electrodes to the generator; a sleeve of insulating material, wherein the scaffold structures are attached to or formed on the sleeve of insulating material and are separated from each other by a distance such that the first and second scaffold structures are electrically insulated from each other.

In the context of a stent, a scaffold is a well understood structure which is capable of supporting the external wall of the vessel into which the stent is placed. A scaffold may be a continuous or substantially continuous piece of material or a framework of interconnected members. It would be understood that the at least one substantially annular portion of each scaffold will have a dimension along the longitudinal axis of the stent (i.e. it will be three dimensional) and that there is, in practice, no limit to the length along this axis. In other words, the scaffold may be substantially annular, including ring-shaped, toroidal and/or cylindrical.

The sleeve of insulating material may cover at least a part of both an inner surface and an outer surface of the scaffold structures. In other words, the scaffold structure may be embedded at least partly within the sleeve with only contact surfaces of the electrodes being exposed.

A further effect of including insulating material on the inner surface of the stent (i.e. between the central portion of the inside of the blood vessel and the electrode) is to promote outward injection of charge rather than inward. This reduces unwanted conduction of signal via the blood, which could lead to short-circuiting, and facilitates a more effective targeting of nerves, which are located outside of the blood vessel. In some embodiments where the scaffold functions as or provides an electrically conductive path, the insulating material may be provided to provide electrical insulation (e.g. to prevent short circuiting) between scaffold structures.

The anodal electrode may also be referred to as a return electrode, and the cathodal electrode may also be referred to as a stimulating electrode.

The cathodal electrode (or the stimulating electrode) may have a surface area of between 0.1 cm² and 0.01 cm², optionally between 0.04 cm² and 0.08 cm², further optionally between 0.05 cm² and 0.075 cm², still further optionally between 0.06 cm² and 0.07 cm², and further optionally 0.067 cm².

**In** a further aspect, the invention provides a stent for intravascular stimulation, the stent comprising: a scaffold formed from an electrically insulating material and comprising at least a first substantially annular portion and a second substantially annular portion spaced apart from the first by a distance; one or more anodal electrodes attached to the first annular portion of the scaffold and one or more cathodal electrodes attached to the second annular portion of the scaffold; wherein each anodal electrode is electrically coupled to an anodal lead to form a conductive path from the respective anodal electrode to a generator and each cathodal electrode is electrically coupled to a cathodal lead to form a conductive path from the respective cathodal electrode to the generator; a sleeve of insulating material, wherein the first and second substantially annular portions are attached to or formed on the sleeve of insulating material.

In a further aspect, the invention provides a stent for intravascular stimulation, the stent comprising: a scaffold formed from a material and comprising at least a first substantially annular portion and a second substantially annular portion spaced apart from the first by a distance; a pulse generator configured to generate electrical signals for delivery to a nerve for intravascular stimulation; one or more anodal electrodes formed from or attached to either the generator or the first substantially annular portion of the scaffold and one or more cathodal electrodes formed from or attached to either the generator or the second substantially annular portion of the scaffold, wherein each anodal electrode and each cathodal electrode is electrically coupled to the pulse generator;
optionally a sleeve of insulating material, wherein the first and second substantially annular portions are attached to or formed on the sleeve of insulating material; and a transducer coupled to the pulse generator and configured to receive energy for delivery of power and/or communications to the pulse generator.

An example of a suitable transducer is an antenna configured to receive EM energy such as RF energy, and convert it into DC power. An alternative suitable transducer is an ultrasonic transducer configured to receive electrical energy and convert it into mechanical energy.

The system may further comprise a battery-operated energiser or charger which may be used to wirelessly power the IPG stimulator via the transducer (e.g. antenna) described above. The powering modality between the charger and the implanted device can be near-field, mid-field, RF or ultrasound. Optionally the battery of the energiser is rechargeable with an external near-field charger.

In some embodiments the stent comprises an energy storage circuit or a battery (for example connected to the pulse generator or the transducer) which can be charged. In other embodiments, the stent may be formed without a battery.

In a further aspect, the invention provides a system for delivery of intravascular stimulation comprising a stent according to any one of above paragraphs and a radio frequency (RF) transmitter configured to transmit RF energy which, when received by the RF antenna of the stent, delivers power and/or communications to the pulse generator of the stent.

In a further aspect, the invention provides a stent structure comprising a scaffold having at least one substantially annular portion comprising one or more hooks or projections, each hook or projection being connected at one end to the substantially annular portion and being unconnected at an opposing end to enable attachment of an electrode to the hook or projection.

In a further aspect, the invention provides a stent for intravascular stimulation, the stent comprising: a scaffold formed from a material and comprising at least a first substantially annular portion and a second substantially annular portion spaced apart from the first by a distance; a pulse generator configured to generate electrical signals for delivery to a nerve for intravascular stimulation; one or more anodal electrodes formed from or attached to either the generator or the first substantially annular portion of the scaffold and one or more cathodal electrodes formed from or attached to either the generator or the second substantially annular portion of the scaffold, wherein each anodal electrode and each cathodal electrode is electrically coupled to the pulse generator;
optionally a sleeve of insulating material, optionally wherein the first and second substantially annular portions are attached to or formed on the sleeve of insulating material; and/or wherein the pulse generator is configured to deliver a signal for intravascular stimulation via the anodal and cathodal electrodes for duration of between 60 seconds and 300 seconds, wherein the signal is formed of a train of pulses, and/or wherein: the pulses have a current amplitudes of between 10mA and 50mA, optionally between 20mA and 40mA; and/or the pulses have a pulse width of between 1ms to 4ms, optionally between 2ms and 3ms; and/or wherein either: A: the pulses have a frequency of between 5Hz and 15Hz, optionally between 8Hz and 12Hz, optionally 10Hz and the pulse train is delivered according to an ON/OFF cycle having a duty cycle of between 10% and 30%, optionally between 15% and 25%, optionally 20%.; or B: the pulses have a frequency of between 0.5Hz and 1.5Hz, optionally 1Hz and the pulse train is delivered continuously.

### Brief description of the figures

Embodiments of the invention will be described, by way of example, with reference to the following drawings, in which:
Figure 1 is an embodiment of an exemplary split-stent-electrode according to the invention;
Figure 2 is a cross-section of an exemplary stent-electrode according to the invention showing an arrangement of contact electrodes;
Figure 3 is a cross-section of three further exemplary stent-electrodes according to the invention showing arrangements of contact electrodes comprising 4, 6 and 8 contacts per anode/cathode;
Figures 4-6 show embodiments of split-stent-electrodes according to the invention each comprising an IPG stimulator and an antenna for receiving power;
Figure 7 is a diagram of an exemplary stent-electrode according to the invention implanted into a patient and in electrical communication with a charger;
Figure 8 is a diagram of a system for providing power to an exemplary stent-electrode according to the invention;
Figures 9 and 10 are time plots showing relationships between current delivered to an exemplary stent-electrode according to the invention and voltage out during setup, stimulation and standby periods;
Figure 11 is a plot showing eCAP data for an exemplary stent-electrode according to the invention;
Figure 12 is a communications and control system according to the invention;
Figure 13 and 14 are diagram of an exemplary stent-electrode according to the invention;
Figures 15A and 15B are modelling of weighting functions and activation functions of potentials at the nerve (at a 1.6mm electrode-fiber distance) of the exemplary stent-electrodes of figures 16 to 22.
Figure 16 shows a two ring electrode stent assuming the scaffold provides a perfect insulator, according to the invention;
Figure 17 shows an alternative two ring electrode stent according to the invention;
Figure 18 shows an alternative two ring electrode stent with 25µm PTFE coating according to the invention;
Figure 19 shows a one ring stent anode according to the invention;
Figures 20 and 21 show two asymmetric split stents according to the invention;
Figure 22 shows a symmetric split stent according to the invention; and
Figure 23 is a graph illustrating results of porcine ex-vivo comparator studies.

### Detailed description

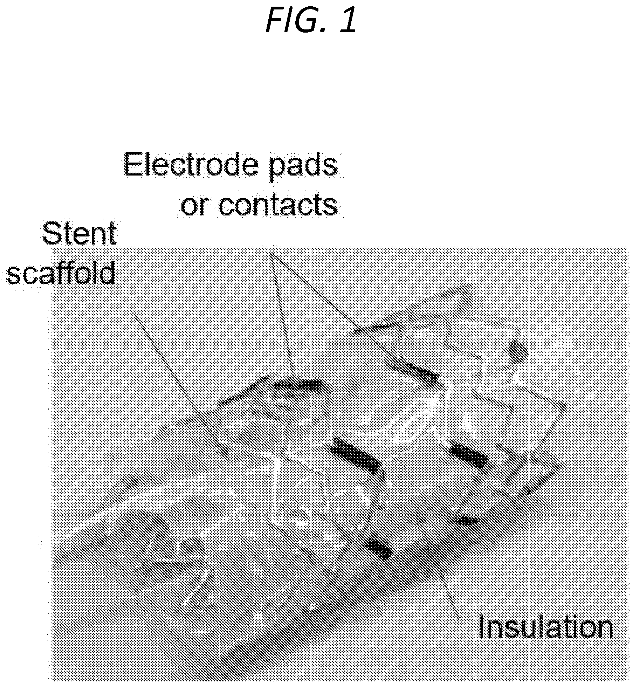

Figure 1 shows an exemplary split-stent-electrode chronic intravascular stimulator according to an aspect of the invention and comprising of an anode and cathode built on a scaffold made of 316L stainless steel. As shown, the scaffold is responsible for providing sufficient radial compliance for the stent to withstand the environment within a blood vessel. Accordingly, the scaffold provides sufficient, hoop force, mechanical strength and robustness to achieve that function, as well as to allow for crimping and expansion to ensure chronic lifetime survivability. Whilst the scaffold of the embodiment of figure 1 is made from 316L stainless steel, other forms of stainless steel may be used, as could Nitinol, Cobolt Alloy or other stent material optimized for the purpose described.

The stimulator comprises a plurality of contact electrodes (or pads), which are crimped to the stent scaffold such that a conductive path is formed between the contacts and the stent scaffold. Although the contacts are crimped to the scaffold in the embodiment of figure 1, other means of attachment could be used, such as welding for example the contacts may be laser welded or resistance welded to the scaffold. Other processes for providing mechanical attachment may also be used. In the event that the scaffold is electrically conductive the attachment process is such that electrical continuity is provided between the contacts and the stent scaffold. In the event that the scaffold is non-conductive a separate conductor such as a wire provides electrical continuity between the contacts and the stent scaffold. Alternatively, the contacts could be formed as part of (or all of) the stent scaffold. The contact electrodes comprise an anode and cathode, though this nomenclature may not always be appropriate in all use-cases for the invention - for instance it will be appreciated that in symmetric biphasic stimulation, each electrode will act as both a cathode and an anode. In this situation, either contact electrode will satisfy the requirements for being an anode and a cathode. In some embodiments, monopolar stimulation may be achieved if an implantable pulse generator is provided, wherein a case of the implantable pulse generator is used as one of the electrode or electrodes. In other embodiments where an implantable pulse generator is provided, the electrode (or a plurality of the electrodes) may be provided (or located) on the implantable pulse generator itself. In some embodiments, the scaffold may have an electrode, although there may also be cases in which the scaffold acts only as a structural support with any electrodes being provided (or located) on the IPG.

The contact electrodes of the embodiment of figure 1 are made from platinum though it will be appreciated that other materials may be used in order to achieve the necessary electrical charge injection properties and/or suitability to coating processes that enhance charge storage capacity. For example, the contact electrodes may comprise platinum, or be formed from an alloy of platinum and iridium, such as an alloy made from 90% platinum and 10% iridium. Alternatively or additionally, the surfaces of the contact electrodes may be coated possibly with PEDOT, PEDOT:PTS, TiNi, IrOx, PtBlack or treated using a process of laser roughening.

The scaffold, including the anode and cathode, of the stimulator is attached to a flexible, conforming, insulating material in the form of a sleeve. The sleeve of the embodiment of figure 1 is made from high durometer polyurethane, but other materials may be suitable, such as nylon, polyester, or Pebax. The thickness of the polyurethane may be between 25µm and 50µm.

The attachment or formation between the scaffold structures and the sleeve can be achieved in various ways. For example an insulating sleeve may be provided, and the scaffold may be deposited on the outer surface of the insulating sleeve using a conventional material deposition process. Instead of deposition, the scaffold may be formed separately and attached to the sleeve by any conventional process such as adhesive or welding. Alternatively, the scaffold and the sleeve may be manufactured together using, for example, an additive manufacturing process. Alternatively it is possible to manufacture the scaffold structure and then form the sleeve on the outer surface of the scaffold structure, for example by depositing, overmolding, overlaying or otherwise placing the sleeve on the scaffold structure. In this case, it may be preferable to remove material from the sleeve to reveal parts of the scaffold structure such as the electrodes. Removing material in this way could be achieved from an etching or laser machining process, for example.

As shown in figure 1, the scaffold is formed in two parts - or structures - which are spaced apart from each other and thus electrically insulated from each other by virtue of the insulating sleeve. The or each cathode contact electrode is provided on one scaffold structure, whilst the or each anode contact electrode is provided on the other scaffold structure. Thus, providing each scaffold structure is formed from an electrically conductive material, it will be appreciated that this aspect of the invention may be performed by electrically connecting each scaffold structure to an implantable pulse generator (discussed elsewhere herein) at a single point, and forming the electrical path from the implantable pulse generator to each contact electrode via the scaffold structure. In other words, electrical contact to each exposed electrode can be achieved by welding or crimping a conductive insulated wire assembly directly onto the stent scaffold and not to each individual electrodes, thereby minimizing the number of welding points to improve the overall robustness of the stent-electrode design. In other embodiments, a plurality of implantable pulse generators may be provided to electrically connect the scaffold structures at a single or multiple points.

The arrangement of the scaffold structures and insulating sleeve is such that the anodal and cathodal contact electrodes do not short circuit each other. In other words, it will be appreciated that a portion of the insulating sleeve defines the inter-electrode separation between the anode and cathode.

In various embodiments of the invention, the insulating material of the sleeve may encapsulate the entirety of the stent scaffold, leaving only the outwardly facing electrode surfaces exposed towards the endothelial tissue to stimulate the nerves around the artery (or other vessel wall). In such embodiments, it will be appreciated that the total electrode area is determined by the sum of the total exposed electrode material and not the totality of the stent scaffolding material of the corresponding anode or cathode. This allows a degree of freedom and control in the design of the stimulator that allows somewhat independent optimization of its mechanical and electrical functions.

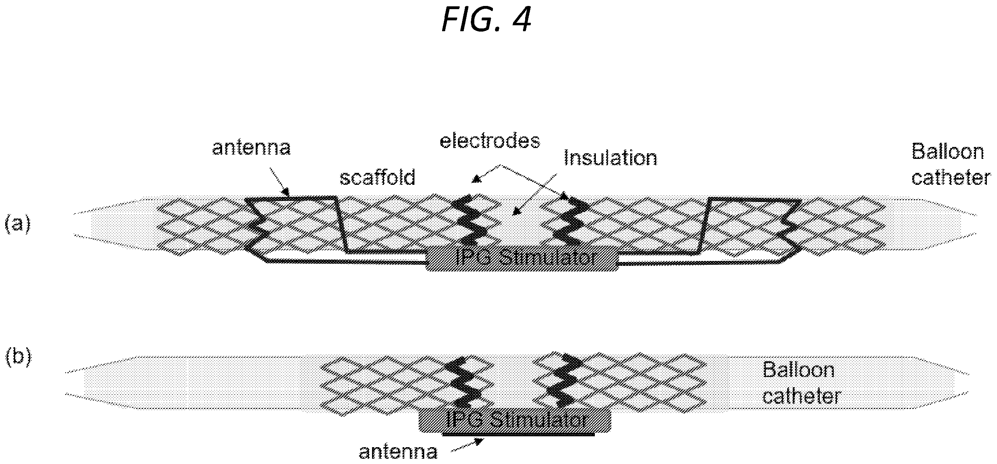

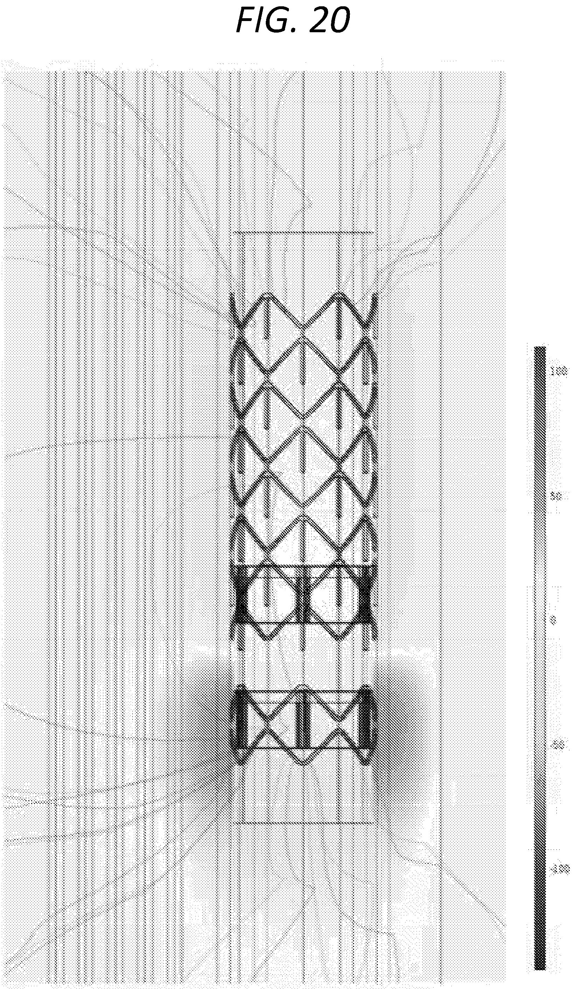

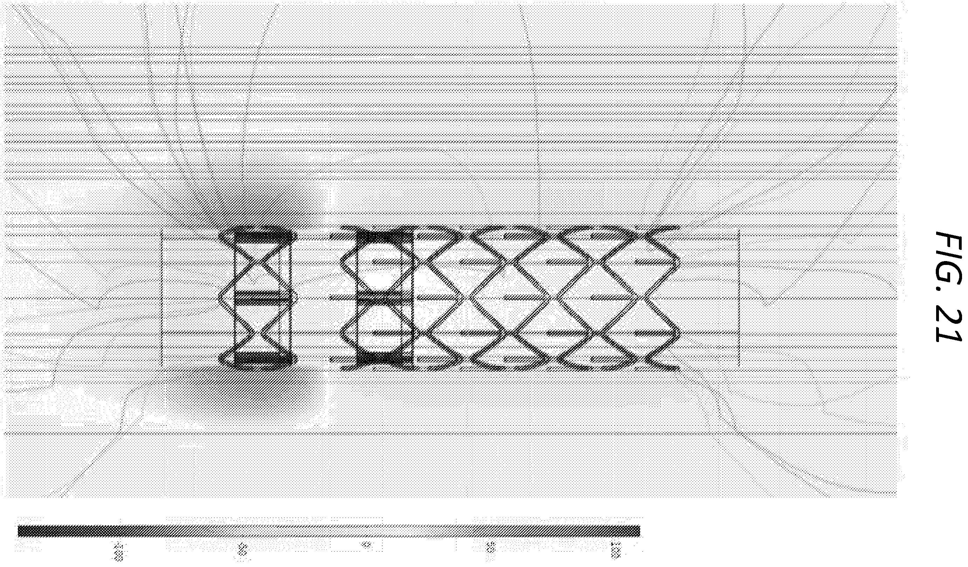

Other embodiments of split-stent-electrode chronic intravascular stimulator operating on the principles described above are shown in figures 4 to 6 and 20 to 22.

Whilst it will be appreciated that an advantage of the split-stent type is that the scaffold itself is conductive, the scaffold may also be non-conductive. In the event that the scaffold is non-conductive the IPG may be used to bridge the split parts of the stent, both mechanically and electrically.

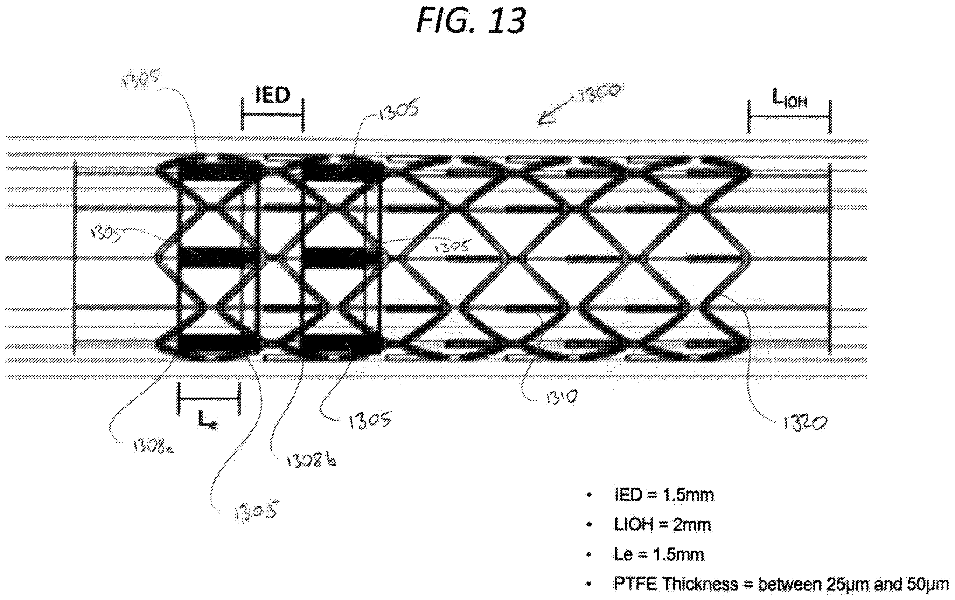

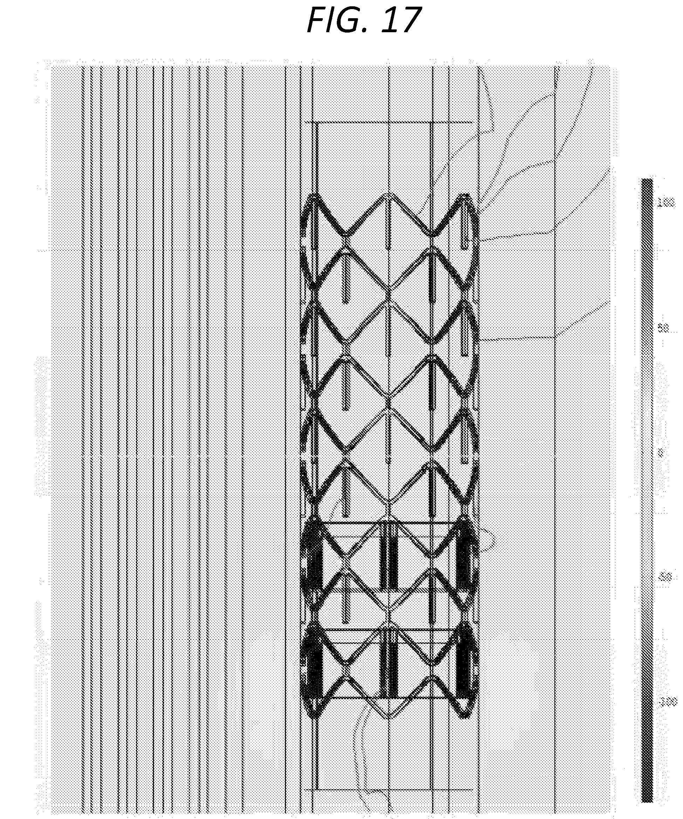

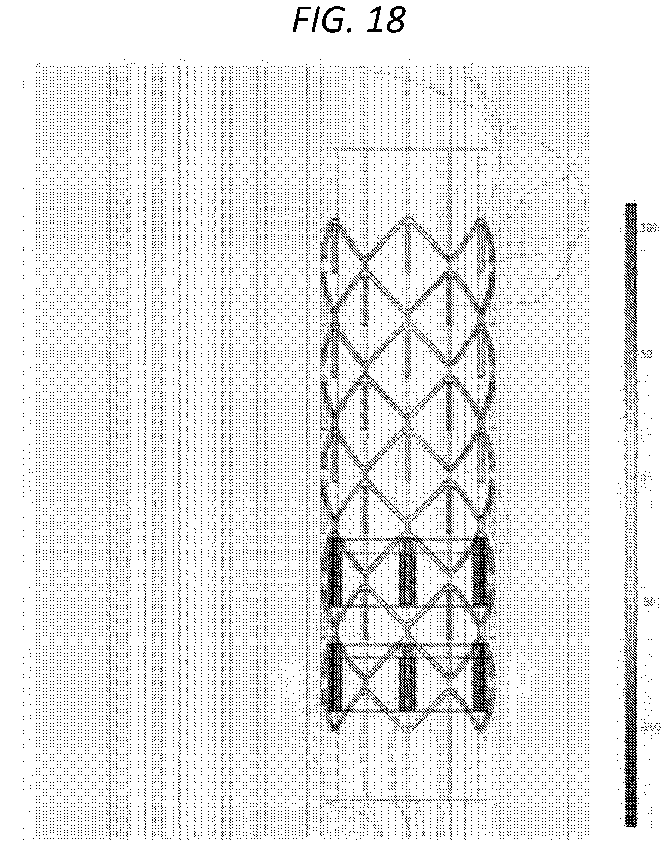

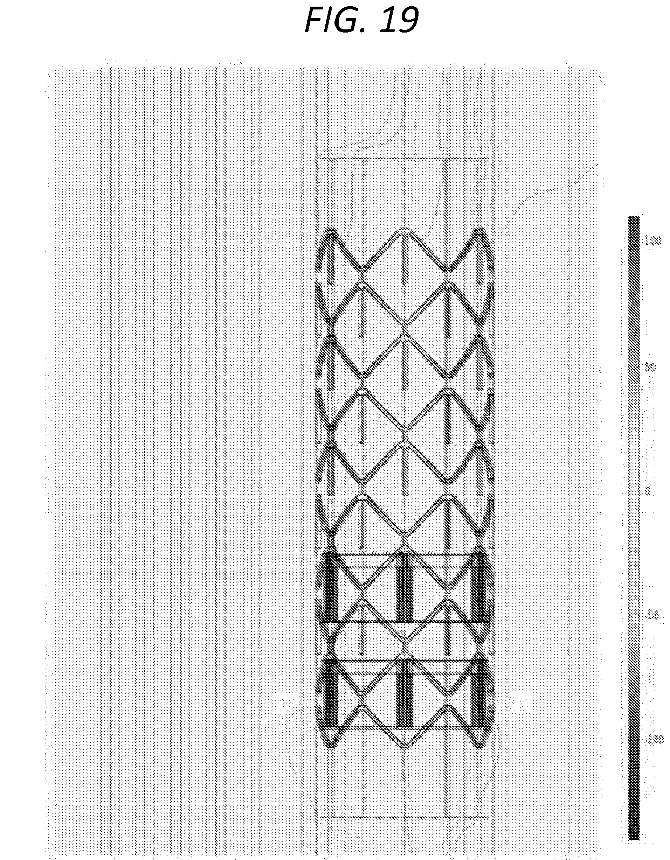

Exemplary embodiments of stent-electrode chronic intravascular stimulators according to the invention which are not of the split-stent type are shown in figures 13 and 16 to 19. Such embodiments also comprise an anode and cathode, but are instead built on a non-conductive scaffold. In these embodiments, the scaffold is made of biocompatible stable polymer, but other non-conductive scaffold material may be used instead so long as the scaffold provides sufficient, hoop force, mechanical strength and robustness to achieve its function, as well as to allow for crimping and expansion to ensure chronic lifetime survivability.

It will be appreciated that the embodiments of stimulators shown in figures 13 and 16 to 19, the scaffolds of which are not conductive, have electrodes that are crimped rather than welded onto the scaffold, though other means of attachment such as bonding could be used instead. Moreover, it will be appreciated that each electrode contacts is electrically contacted by welding or crimping to an insulated wire assembly connectable to an implantable signal generator (discussed elsewhere herein). In some embodiments the IPG may be passive; that is, for example, not powered by its own power source such as a battery. In such cases, a transducer may be provided on the implantable device for receiving signals from an external pulse generator, which are then communicated to the electrodes to deliver the signals. As described elsewhere herein, the transducers may be configured to receive whichever form of energy is preferred, such as EM, including RF, or ultrasonic, and converting such signals to electrical signals for delivery to a nerve.

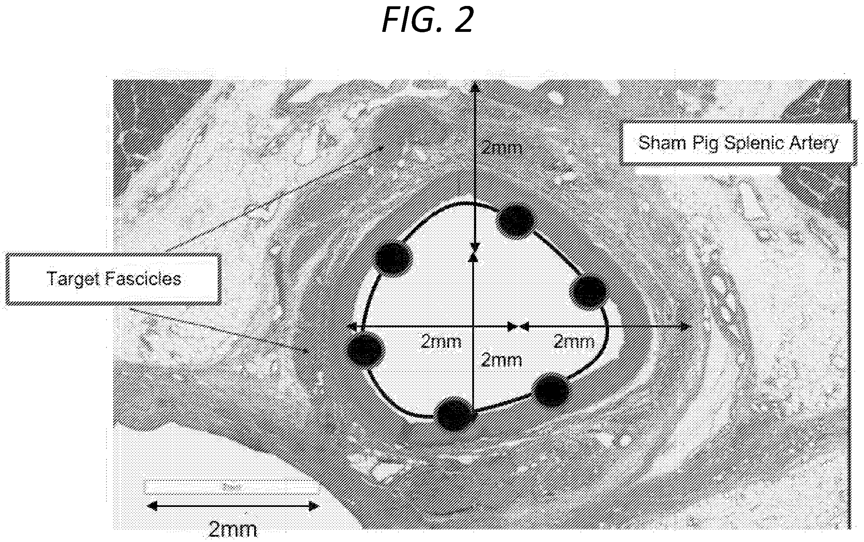

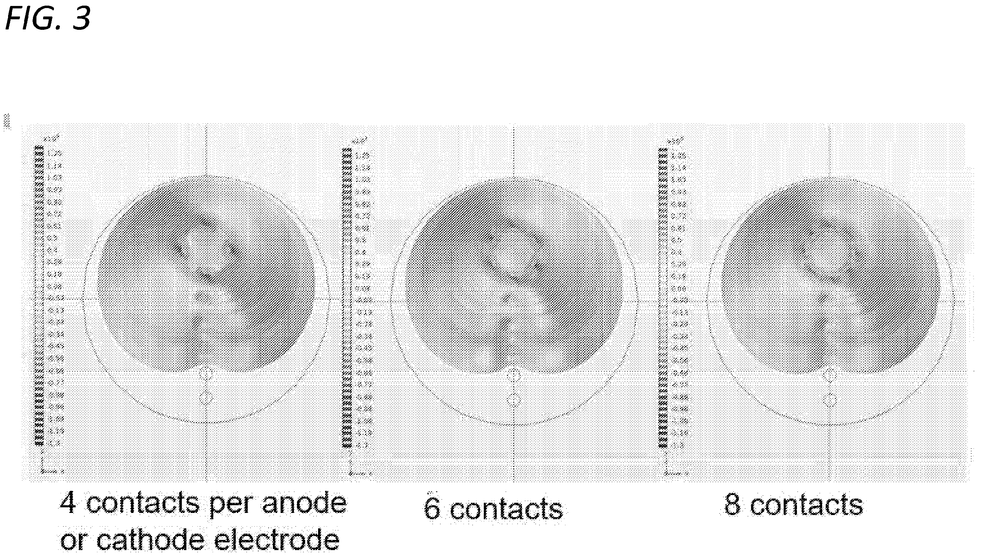

As shown in figures 1 to 3 and elsewhere, the stent-electrode chronic intravascular stimulators (both the split-stent and non-conductive types) may comprise multiple electrode contacts per anode and cathode. In figure 2, six electrode contacts per anode and cathode are shown, but the number of contacts can vary between 4 and 12 depending of the target vessel size. Figure 3 shows alternative embodiments where 4, 6 and 8 electrodes are used. Optionally, 4, 6, 8, 10 or 12 electrode contacts per anode and cathode are used, but 5, 7, 9 or 11 electrode contacts per anode and cathode may be used instead. It is possible for different numbers of electrode contacts to be used for the anode and the cathode, but it is preferred to use the same number for both the anode and the cathode.

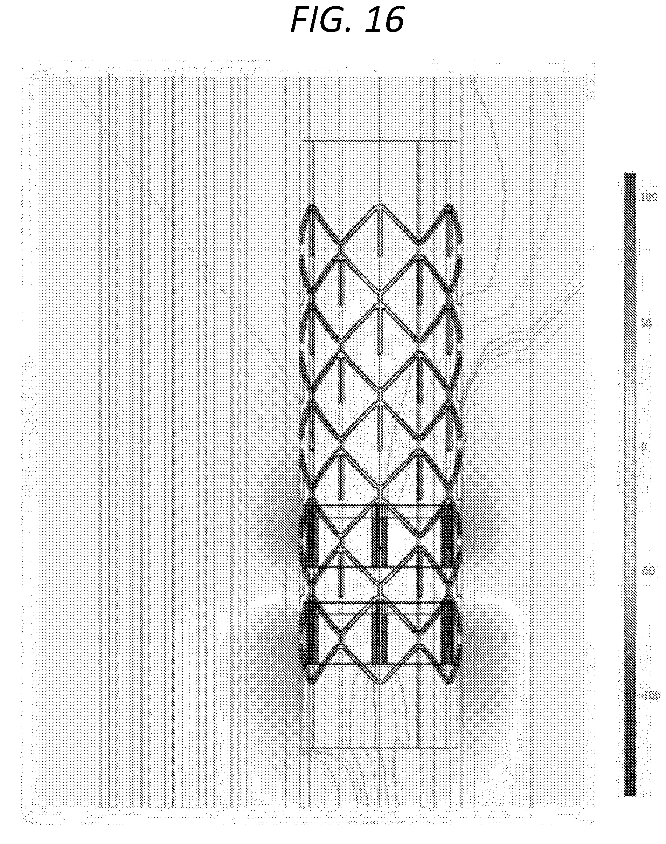

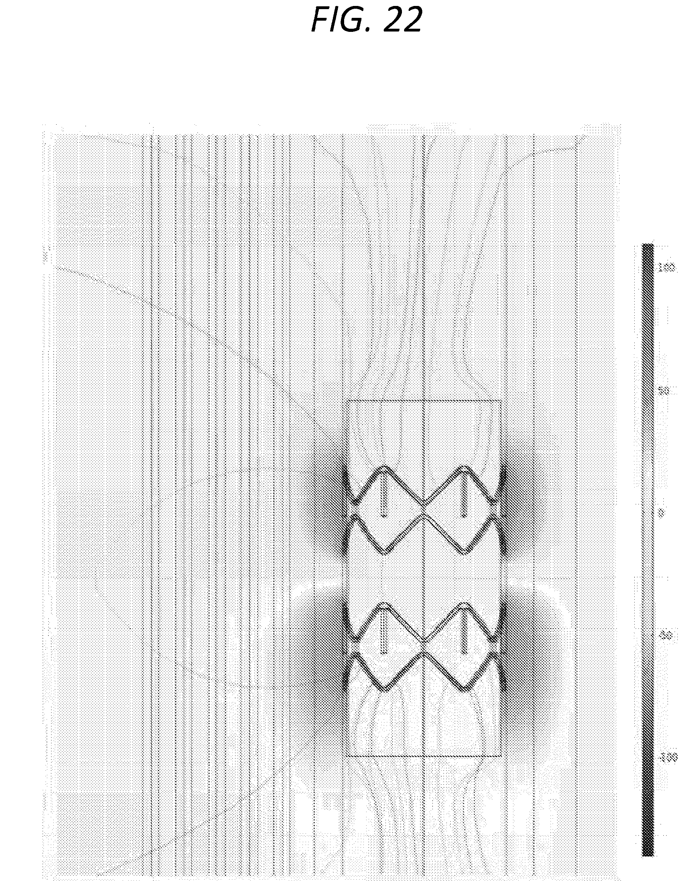

Referring to figures 16 to 22, embodiments according to the invention may be implemented using a symmetric or an asymmetric scaffolding configuration. A symmetric design is typically more compact, but asymmetry may be helpful to facilitate placement and to robustly anchor the device onto a target site. Figure 22 is an example of a symmetric configuration wherein the sets of electrodes are the same distance away from the respective ends of the scaffold and sleeve of insulating material. Figures 16 to 21 are examples of an asymmetric configuration wherein one of the sets of electrode contacts (in this case the anode, with blue indication corresponding to the more negative of the two values indicated on the accompanying measurement scale) is closer to one end of the scaffold and sleeve of insulating material than the other of the sets (in this case the cathode, with red indication corresponding to the more positive of the two values indicated on the accompanying measurement scale) is to the other end. Symmetric and asymmetric embodiments may also exist with a unitary body. The symmetric scaffolding configuration described above may exist as an asymmetric embodiment, and the asymmetric scaffolding configuration described above may exist as a symmetric embodiment.

Figures 4 to 8 show an embodiment of a split-stent-electrode chronic intravascular stimulator comprising a miniature implantable pulse generator (IPG) with wireless antenna for receiving power and communication from a transmitter (described elsewhere herein). Figures 4 to 8 show a split-stent, though the embodiment would be just as applicable to a non-conductive type.

As shown in figures 4 to 6, the embodiments comprises two scaffold structures each including contact electrode(s) as described elsewhere herein. One scaffold structure provides an anode and the other provides a cathode. Each contact electrode is coupled to a miniature IPG in any manner as described elsewhere herein.

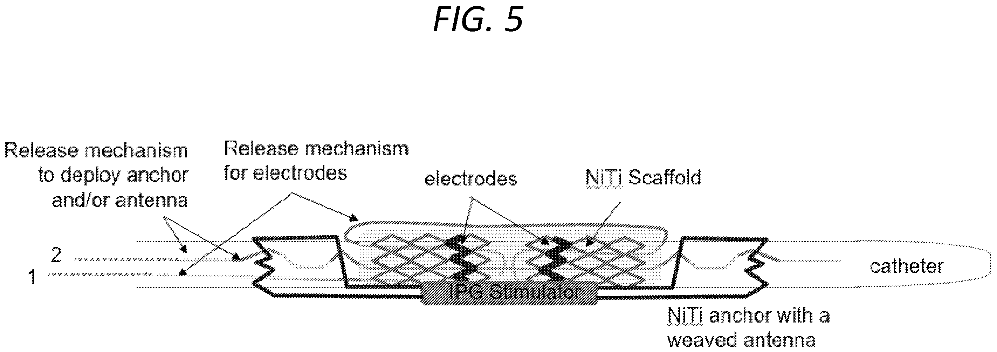

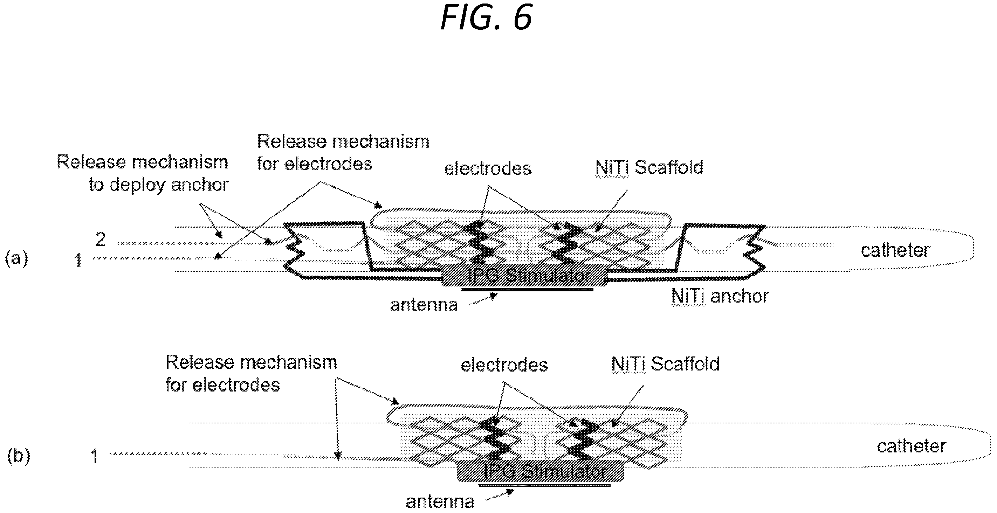

Figure 4 show two versions of a wireless antenna for receiving power and communication from a transmitter. In figure 4a, the antenna is made of electrically insulated multi-conductor weaved in the stent scaffold and attached to the IPG through hermetic feedthrus. In figure 4b the antenna is encapsulated within the IPG stimulator itself. Figures 5 and 6 show an alternative arrangement wherein the antenna is made of electrically insulated multi-conductor weaved with an anchor (described below). The embodiment illustrated in figure 4b may use ultrasound as the powering modality in addition to, or alternative to those that require an antenna (such as RF). For example, the IPG stimulator may comprise a transducer for delivering electrical energy to the electrodes using energy source such as ultrasound. In other words, some embodiments do not require an antenna.

It will be appreciated that the stimulator may be delivered into the target location of the splenic artery by a balloon expandable catheter. Alternatively or in addition, the stimulator may be made of a self-expanding Nitinol scaffold and comprise an anchor, and be delivered to the target location of the splenic artery by a selective release mechanism. In the examples shown in figures 5 and 6, the electrodes can be partially deployed and retracted using a wire pully mechanism ("1") that may comprise of one or two wires controlled proximally by the operator. Partial deployment of the electrodes may be used to determine the optimal placement of the device intraoperatively. A second deployment mechanism may be used to anchor the device in the vessel using a NiTi anchor released by retracting a wire holding the anchor in place in its collapsed state. This mechanism allows for a controlled release.

Figure 6a shows a self-expandable device with the same controlled release mechanism described above, wherein the antenna is embedded with the IPG but retaining a retractable NiTi anchor as described above. Figure 6b shows a further embodiment wherein the antenna is embedded with the IPG stimulator and anchoring to the artery is accomplished using the NiTi stent scaffold itself. In this case, the scaffold can be made of an expandable insulative polymer matrix. The embodiment illustrated in figure 6b may use ultrasound as the powering modality in addition to, or alternative to those that require an antenna (such as RF). For example, the IPG stimulator may comprise a transducer for delivering electrical energy to the electrodes using energy source such as ultrasound.

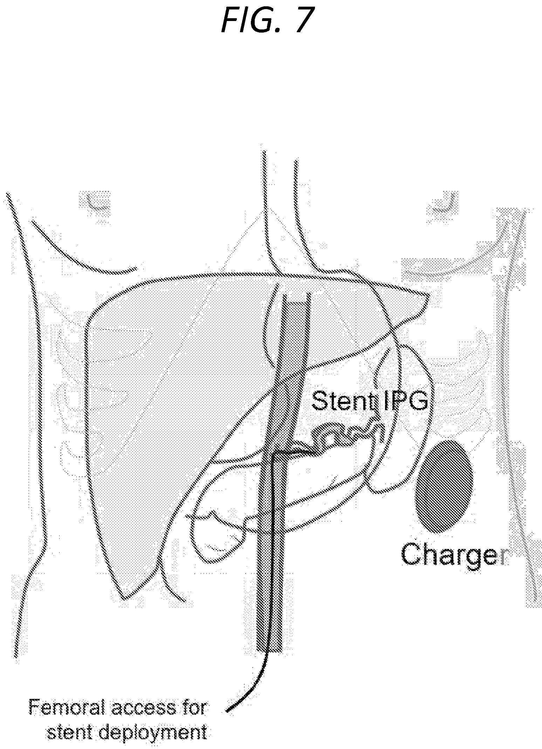

Figure 7 illustrates a stent as described elsewhere herein as part of a system according to an aspect of the invention. The system comprises the stent-electrode stimulator, which is shown in figure 7 as deployed into the splenic artery through femoral access using a 7Fr to 9Fr delivery system. The system further comprises battery-operated energizer or charger which is used to wirelessly power the IPG stimulator via the antenna described above. The powering modality between the charger and the stent-electrode IPG stimulator can be near-field, mid-field or ultrasound. Optionally the battery of the energizer is rechargeable with an external near-field charger.

The energizer (and optionally its charger) may be a wearable device, or may be implanted in a subcutaneous pocket of a patient. A wearable device may be advantageous for ad-hoc stimulation and/or where the charger requires frequent recharging. Conversely, an implantable device may be advantageous to deliver continuous stimulation, or deliver a scheduled therapy on a program, wherein the powering modality between the implanted charger and the IPG stimulator can be near-field, mid-field or ultrasound.

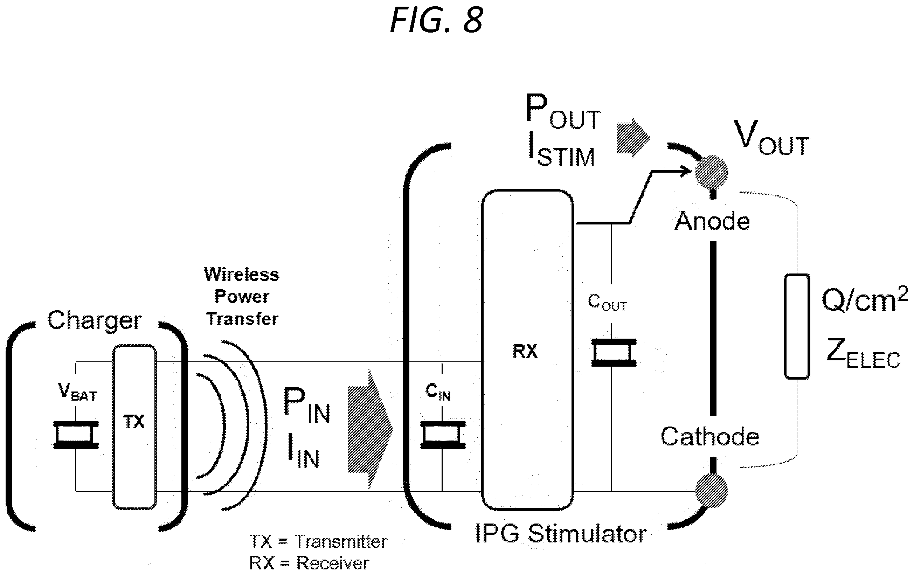

Figure 8 shows a power system for the devices described in connection with figure 7. As described in more detail below, energy supplied by the charger is stored and accumulated within the IPG in storage elements such as super-capacitors, and subsequently used to apply therapy.

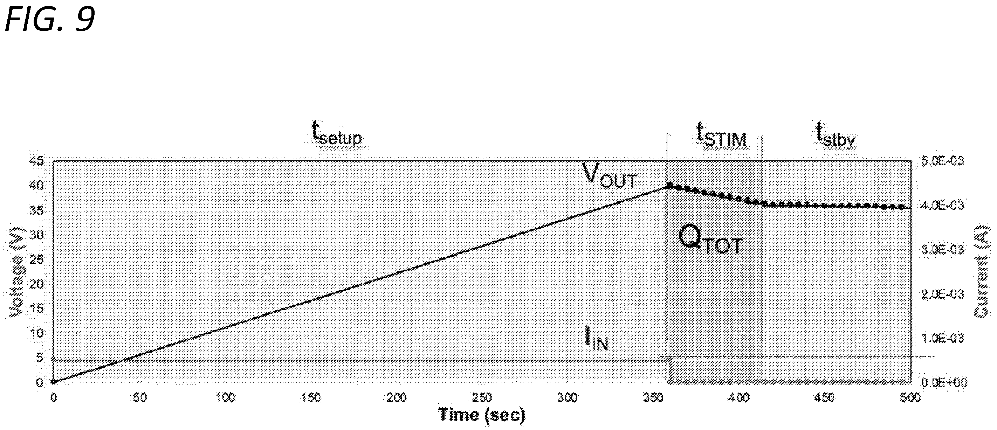

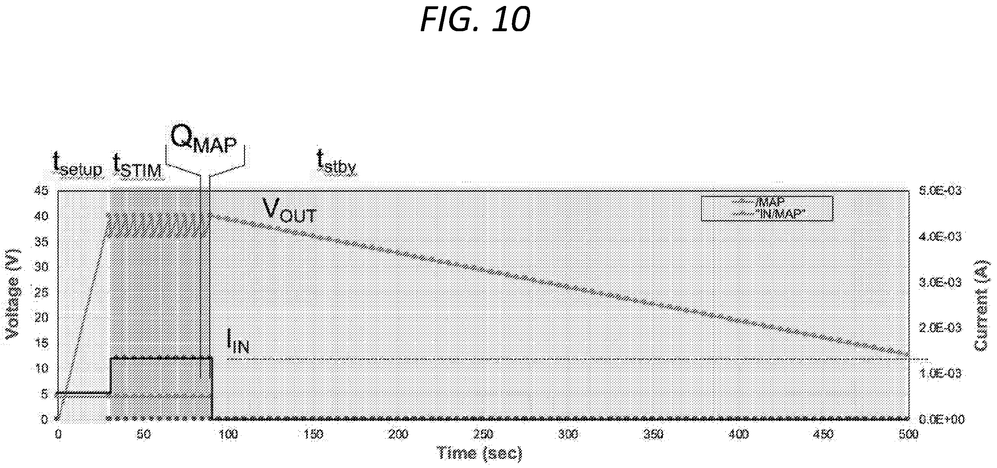

Figure 9 shows an energy transfer schedule applicable to the system of figures 7 and 8. In this case, energy supplied from a wireless charger is stored in capacitors until the total therapeutic dose is accumulated, at which time it is used to apply therapy. Compared with the system of figure 10, this system requires larger capacitors and a longer time to accumulate the charge for stimulation. On the other hand this system requires a lower input energy to 'trickle-charge' the capacitor or other storage elements to the desired output voltage level. Moreover, it only requires energy to be supplied continuously until the required voltage is reached, at which point the charger can cease delivery of energy.

Figure 10 shows an alternative energy transfer schedule applicable to the system of figures 7 and 8. Again, in this case energy supplied from a wireless charger is stored in capacitors until the charge required to deliver a micro-burst is accumulated. In particular, this is the charge required to deliver an 'active period' dose comprising of a burst of pulses in one active period. Once the required charge is accumulated, it is used to apply therapy. Compared with the system of figure 9, this system requires smaller capacitors, and less time to accumulate the charge for stimulation. On the other hand, the system requires slightly higher input energy to charge the capacitor or other storage elements to the desired output voltage level. Moreover, it requires energy supplied continuously through the duration of the therapy.

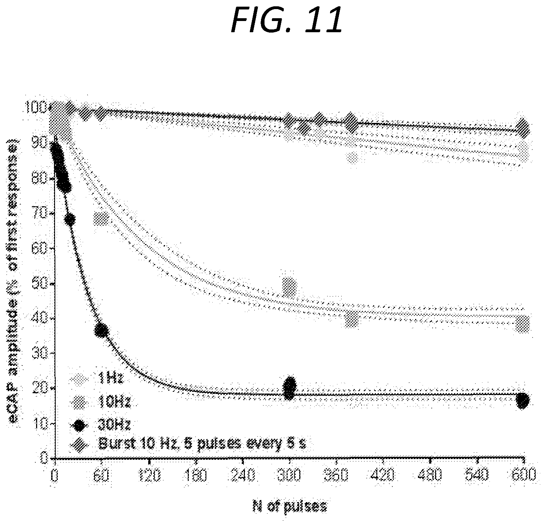

Figure 11 shows eCAP amplitude (in percentage of first responses) over number N of pulses for different signal parameters, specifically 1Hz, 10Hz, 30Hz continuous, and a burst pattern of 10Hz comprising 5 pulses every 5 seconds. In one embodiment, preferred parameters of stent-electrode stimulation systems described elsewhere herein are 10Hz pulses with 0.5sec ON time and 5sec OFF time at current amplitudes ranging from 10mA to 40mA and pulse widths ranging from 1ms to 4ms, for a total duration of 60sec up to 300sec. In another embodiment, preferred parameters of stent-electrode stimulation systems described elsewhere herein are 1Hz pulses delivered continuously at current amplitudes ranging from 10mA to 40mA and pulse widths ranging from 1ms to 4ms, for a total duration of 60sec up to 300sec. This will achieve the same therapeutic effect as 10Hz, 0.5sec/5sec ON/OFF parameters but with less peak input energy demands.

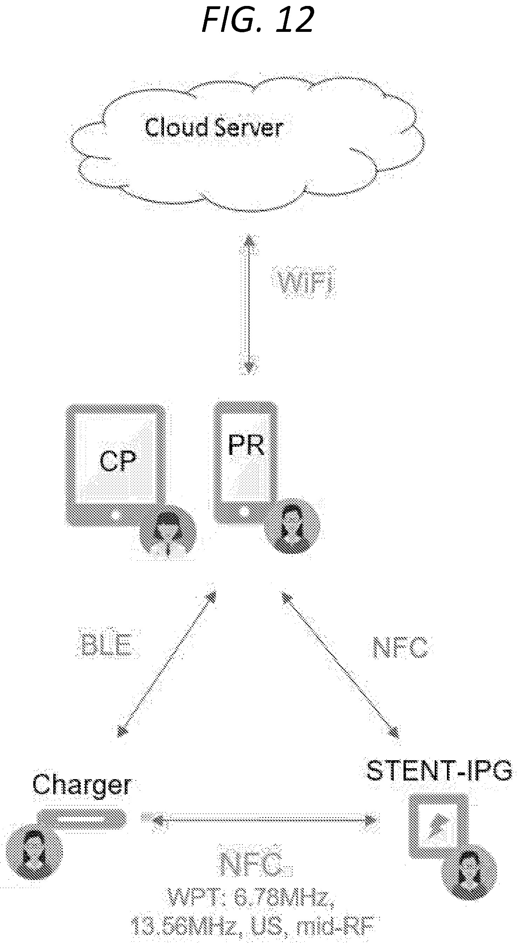

Figure 12A shows a communications and control system according to the invention. The system comprises the stent-IPG stimulator and charger described elsewhere herein, and a deployment catheter. It also comprises a patient remote (PR) and clinician programmer (CP).

In a preferred embodiment, the CP connects to the charger via BLE to program therapy parameters in non-volatile memory. Therapy parameters may also be programmed into the stent IPG non-volatile memory. The CP & PR are used to monitor therapy by communicatively coupling with the charger while the charger energizes the IPG stent implant to deliver therapy.

In the illustrated embodiment, the CP/PR software applications serve as the gateway to cloud connectivity and are used to download therapy parameters, track compliance and send patient reminders. Additionally the CP/PR can connect directly to the stent implant via NFC link for diagnostics or monitoring purposes.

In the illustrated embodiment, the charger energizes the stent-IPG through wireless powering scheme based on 6.78MHz or 13.56MHz ISM bands, ultrasound or mid-field powering. It supports BLE with the CP/PR and NFC with the stent IPG. It will be appreciated that other wireless powering schemes may be used, and other communication protocols may be used for communication.

In some embodiments, the stent-IPG stimulator is energized by the charger while therapy is being delivered. It may also link to external devices such as the charger and CP/PR over the NFC protocol. The stent-IPG is a single-fault safe device since it does not contain a battery and is not intended to be explanted in the event of failure.

In summary, the charger of the system of figure 12A energizes the stent-IPG stimulator via NFC to deliver therapy and links to the CP/PR via BLE. The CP/PR of figure 12 provides patient app gateway to the cloud in order to download/upload therapy parameters, track compliance and sends reminders. The CP/PR also links to the charger via BLE to monitor progress during therapy and links to the stent-IPG stimulator via NFC for diagnostics and monitoring. Finally, the stent-IPG stimulator is energized by charger while therapy is delivered and links to Charger or CP/PR via NFC. It is a single-fault safe device, by which it is meant that it does not comprise a battery.

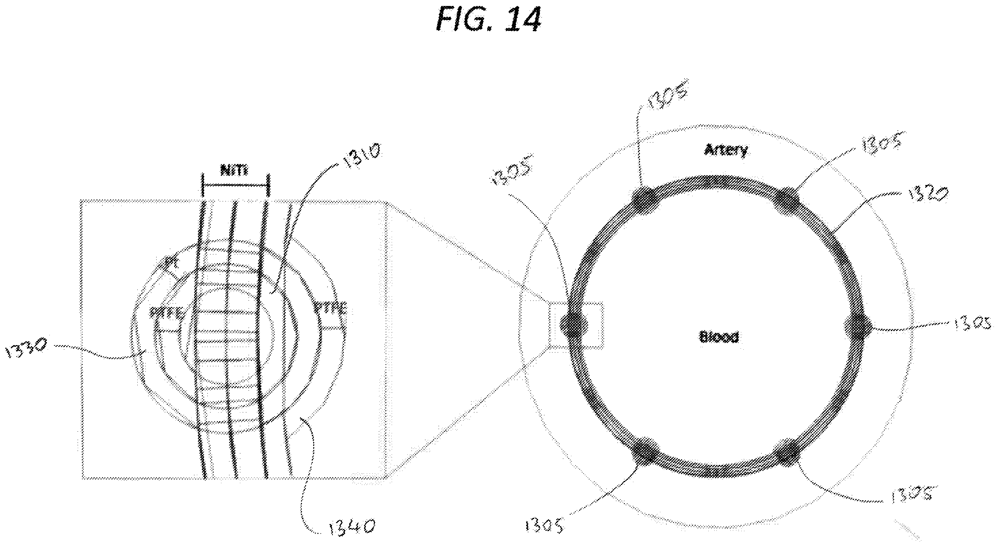

Figures 13 and 14 are diagram of an exemplary stent-electrode 1300 according to the invention. As shown, in this particular example of a stent-electrode 1300, the inter-electrode distance (IED) is 1.5mm, the electrode length (Le) is 1.5mm, the thickness of the PTFE material (see figure 14) is between 25 and 50 µm and the length of insulation (in this case the PTFE insulation film) over-hang (L_{IOH}). Of course, it will be appreciated that these dimensions are merely exemplary, and other dimensions will be suitable depending on the particular application. In the example shown, six electrodes 1305 are equally spaced circumferentially around the perimeter of each substantially annular portion of the stent-electrode 1300, though more or fewer electrodes can be provided, and the spacing can be adjusted depending on the particular application. In the illustrated case, two substantially annular portions 1308a, 1308b are provided, resulting in a total of twelve electrodes.

The stent-electrode 1300 of figures 13 and 14 has features and concepts in common with the devices described above in connection with figures 1 to 12. In addition, the stent-electrode 1300 of figures 13 and 14 is provided with hooks or projections 1310 that, in the illustrated embodiment, extend from the or each substantially annular portion 1308 of the scaffold 1320, and extend along the longitudinal axis of the stent. These hooks or projections 1310 can be any suitable shape, and are for the purpose of attaching or forming the electrodes 1305. As shown in figure 13, each hook or projection 1310 of the relevant substantially annular portion 1308a, 1308b carries a respective electrode 1305, though it is not necessary for every hook or projection 1310 of the scaffold 1320 to carry an electrode.

Figure 14 shows the cross section of the stent-electrode in figure 13. The scaffold 1320 of the stent-electrode has a circular cross section, and is made from Nitinol (i.e. an alloy of nickel and titanium). Each hook or projection 1310 is has an annular cross-section, though the feature need not be hollow, and could take other cross-sectional shapes. Each hook or projection 1310 may be formed in the same material as the rest of the scaffold or be made from polytetrafluoroethylene (PTFE), though other insulating materials could be used instead. Such insulating materials can be provided at least partly over the hook to provide an increased mechanical attachment between the scaffold hook and the electrode layer or electrode coating, for example the platinum layer 1330. In an embodiment where the scaffold provides an electrically conductive path, at least a part of the electrode layer 1330 is in mechanical and electrical contact with the scaffold hook. In some embodiments, insulating material may not be provided between the scaffold hook 1310 and the platinum layer 1330. Surrounding each hook or projection 1310 is a platinum layer 1330, which forms the electrode 1305 itself. It will be appreciated that in the illustrated embodiment where the stent-electrode scaffold 1320 is conductive, an electrical signal may pass from the scaffold 1320 to the electrode 1305, specifically to the platinum layer 1330. Any suitable electrical connection between these two components will facilitate this electrical coupling. In cases where the scaffold is not conductive, a separate electrical connection, for example a wire, may couple directly to the electrode, for example to a platinum layer similar to the one illustrated.

To prevent an electrical signal passing from the platinum layer toward the inside of the vessel rather than toward the vessel wall as intended, a PTFE coating 1340 is provided over the portion of the platinum layer that is positioned internal to the scaffold 1320; i.e. the portion of the platinum layer that faces radially inwardly. It will be noted that there is no such PTFE coating 1340 provided over the portion of the platinum layer that is positioned external to the scaffold 1320; i.e. the portion of the platinum layer that faces radially outwardly. It will be appreciated that this arrangement promotes outward injection of charge rather than inward, which reduces unwanted conduction of signal via the blood, as described above.

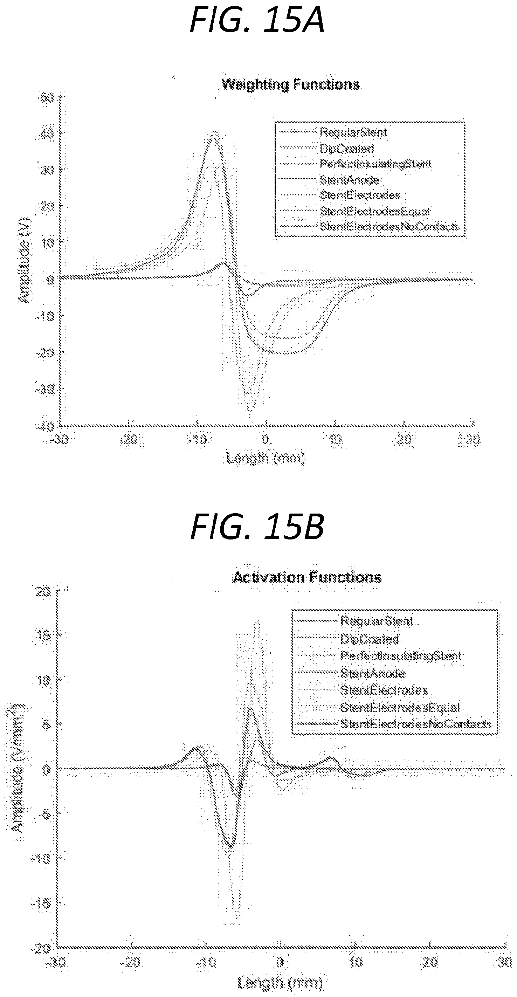

Figures 15A and 15B show modelling of weighting functions and activation functions of potentials at the nerve for different electrode designs and configurations (for example those illustrated in Figures 16-23). The activation function is the second spatial derivative of the electric field, and it allows determination of the location(s) on the electrode where the system will cause greatest activation of, for example, the nerve. This enables determination of factors such as the number, positioning, spatial separation, and size of electrodes. The activation function aids selection of the ideal electrode design and configuration.

Figures 16 to 23 show various examples of six-electrode stents of one-ring, two-ring and split-stent design employing aspects of the invention described above.

The stent may be positioned inside the splenic artery for stimulating the splenic nerve or branches of the splenic nerve.

The stent of this application could be used in conjunction with any suitable blood vessel in order to apply an electrical signal to any corresponding nerve. Other examples include: the carotid artery and the vagus nerve and the cervical sympathetic ganglion; the aorta and the phrenic nerve, the vagus nerve, the superior mesenteric ganglion, and the inferior mesenteric ganglion; the renal artery and the renal nerves; and the subclavian artery and the brachial plexus; and common hepatic artery and its associated nerves; and gastroduodenal artery and its associated nerves; iliac artery and splanchnic nerves.

The invention may be useful for treating subjects who are suffering from, or who are at risk of developing, diseases, disorders or conditions associated with inflammation, e.g. inflammatory disorders, e.g., autoimmune disorders. The invention may treat or ameliorate the effects of such diseases, disorders or conditions by reducing inflammation. This may be achieved by decreasing the production and release of pro-inflammatory cytokines, and/or by increasing the production and release of anti-inflammatory cytokines and pro-resolving molecules, from the spleen, by electrically stimulating the splenic arterial nerve as described herein.

Inflammatory disorders include autoimmune disorders, such as arthritis (e.g. rheumatoid arthritis, osteoarthritis, psoriatic arthritis), Grave's disease, myasthenia gravis, thryoiditis, systemic lupus erythematosus, Goodpasture's syndrome, Behcets's syndrome, allograft rejection, graft-versus-host disease, ankylosing spondylitis, Berger's disease, diabetes including Type I diabetes, Reitier's syndrome, spondyloarthropathy psoriasis, multiple sclerosis, Inflammatory Bowel Disease, Crohn's disease, Addison's disease, autoimmune mediated hair loss (e.g., alopecia areata) and ulcerative colitis.

Certain examples of inflammatory disorders include diseases involving the gastrointestinal tract and associated tissues, such as appendicitis, peptic, gastric and duodenal ulcers, peritonitis, pancreatitis, ulcerative, pseudomembranous, acute and ischemic colitis, inflammatory bowel disease, diverticulitis, cholangitis, cholecystitis, Crohn's disease, Whipple's disease, hepatitis, abdominal obstruction, volvulus, post-operative ileus, ileus, celiac disease, periodontal disease, pernicious anemia, amebiasis and enteritis.

Examples of inflammatory disease, disorders or conditions affecting the bones, joints, muscles and connective tissues include the various arthritides and arthralgias, osteomyelitis, gout, periodontal disease, rheumatoid arthritis, spondyloarthropathy, ankylosing spondylitis and synovitis.

Further examples include systemic or local inflammatory diseases and conditions, such as asthma, allergy, anaphylactic shock, immune complex disease, sepsis, septicemia, endotoxic shock, eosinophilic granuloma, granulomatosis, organ ischemia, reperfusion injury, organ necrosis, hay fever, cachexia, hyperexia, septic abortion, HIV infection, herpes infection, organ transplant rejection, disseminated bacteremia, Dengue fever, malaria and sarcoidosis.

Other examples include diseases involving the urogential system and associated tissues, such as diseases that include epididymitis, vaginitis, orchitis, urinary tract infection, kidney stone, prostatitis, urethritis, pelvic inflammatory bowel disease, contrast induced nephropathy, reperfusion kidney injury, acute kidney injury, infected kidney stone, herpes infection, and candidiasis.

Other examples include involving the respiratory system and associated tissues, such as bronchitis, asthma, hay fever, ventilator associated lung injury, cystic fibrosis, adult respiratory distress syndrome, pneumonitis, alvealitis, epiglottitis, rhinitis, achlasia, respiratory syncytial virus, pharyngitis, sinusitis, pneumonitis, alvealitis, influenza, pulmonary embolism, hyatid cysts and/or bronchiolitis.

Further examples are dermatological diseases and conditions of the skin (such as bums, dermatitis, dermatomyositis, burns, cellulitis, abscess, contact dermatitis, dermatomyositis, warts, wheal, sunburn, urticaria warts, and wheals); diseases involving the cardiovascular system and associated tissues, (such as myocardial infarction, cardiac tamponade, vasulitis, aortic dissection, coronary artery disease, peripheral vascular disease, aortic abdominal aneurysm, angiitis, endocarditis, arteritis, atherosclerosis, thrombophlebitis, pericarditis, myocarditis, myocardial ischemia, congestive heart failure, periarteritis nodosa, and rheumatic fever, filariasis thrombophlebitis, deep vein thrombosis); as well as various cancers, tumors and proliferative disorders (such as Hodgkin's disease), nosocomial infection; and, in any case the inflammatory or immune host response to any primary disease.

Other examples of inflammatory disorders include diseases involving the central or peripheral nervous system and associated tissues, such as Alzheimer's disease, depression, multiple sclerosis, cerebral infarction, cerebral embolism, carotid artery disease, concussion, subdural hematoma, epidural hematoma, transient ischemic attack, temporal arteritis, spinal cord injury without radiological finding (SCIWORA), cord compression, meningitis, encephalitis, cardiac arrest, Guillain-Barre, spinal cord injury, cerebral venous thrombosis and paralysis.

Inflammatory disorders also include conditions associated with immune or inflammatory response (i.e. acute inflammatory episodes) include injury to nerves or other tissue and pain associated with nerve or other tissue. Injury may be due to a physical, chemical or mechanical trauma. Non- limiting examples of injury include acute trauma, burn, whiplash, musculoskeletal strains, and post-operative surgery complications, such as DVT, cardiac dysrhythmia, ventilator associated lung injury, and post-operative ileus.

Conditions associated with a particular organ such as eye or ear may also include an immune or inflammatory response such as conjunctivitis, iritis, glaucoma, episcleritis, acute retinal occlusion, rupture globe, otitis media, otitis externa, uveitis and Meniere's disease. Another example of an inflammatory disorder is post-operative ileus (POI). POI is experienced by the vast majority of patients undergoing abdominal surgery. POI is characterized by transient impairment of gastro-intestinal (GI) function along the GI tract as well pain and discomfort to the patient and increased hospitalization costs.

The impairment of GI function is not limited to the site of surgery, for example, patients undergoing laparotomy can experience colonic or ruminal dysfunction. POI is at least in part mediated by enhanced levels of pro-inflammatory cytokines and infiltration of leukocytes at the surgical site. Neural inhibitory pathways activated in response to inflammation contribute to the paralysis of secondary GI organs distal to the site of surgery. Stimulation of neural activity as taught herein may thus be effective in the treatment or prevention of POI.

The invention is particularly useful in treating autoimmune disorders (e.g. rheumatoid arthritis, osteoarthritis, psoriatic arthritis, spondyloarthropathy, ankylosing spondylitis, psoriasis, systemic lupus erythematosus (SLE), multiple sclerosis, Inflammatory Bowel Disease, Crohn's disease, and ulcerative colitis) and sepsis.

This invention is particularly useful for treating B cell mediated autoimmune disorders (e.g. systemic lupus erythematosus (SLE) and rheumatoid arthritis (RA)).

The invention is particularly useful for treating inflammatory conditions associated with bacterial infections. For example, the invention is particularly useful for treating inflammatory conditions caused or exacerbated by Escherichia coli, Staphylococcus aureus, Pneumococcus, Haemophilus influenza, Neisseria meningitides, Streptococcus pneumonia, Methicillin-resistant Staphylococcus aureus (MRSA), Klebsiella or Enterobacter infection. Treatment of the inflammatory disorder can be assessed in various ways, but typically involves determining an improvement in one or more physiological parameters of the subject.

Useful physiological parameters may be one or more of the group consisting of: the level of a pro-inflammatory cytokine, the level of an anti-inflammatory cytokine, the level of a catecholamine, the level of an immune cell population, the level of an immune cell surface co-stimulatory molecule, the level of a factor involved in the inflammation cascade, the level of an immune response mediator, and the rate of splenic blood flow.

Improvement in a determined physiological parameter in the context of the invention may be one or more of the group consisting of: a reduction in a pro-inflammatory cytokine, an increase in an anti-inflammatory cytokine, an increase in a catecholamine, a change in an immune cell population, a change in an immune cell surface co-stimulatory molecule, a reduction in a factor involved in the inflammation cascade, a change in the level of an immune response mediator and a decrease in splenic blood flow. The invention might not lead to a change in all of these parameters.

By stimulating a splenic arterial nerve at a site where the splenic artery is not in direct contact with the pancreas, the spleen may: (a) decrease the secretion of a pro-inflammatory cytokine compared to baseline secretion; and/or (b) increase the secretion of an anti-inflammatory cytokine compared to baseline secretion. For example, the decrease in a pro-inflammatory cytokine secretion may be by: ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90% or ≤ 95%. The increase in an anti-inflammatory cytokine secretion may be by: ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, ≤ 95%, ≤ 100%, ≤ 150% or ≤ 200%.

Once the cytokine is secreted into the circulation, its concentration in the circulation is diluted. Stimulation of the splenic arterial nerve may result in: (a) a decrease in the level of a pro-inflammatory cytokine in the plasma or serum by ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, or ≤ 95%; and/or (b) an increase in the level of an anti-inflammatory cytokine in the plasma or serum by ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, ≤ 95%, ≤ 100%, ≤ 150% or ≤ 200%. Preferably the level in the serum is measured. By stimulating the splenic arterial nerve, the level of catecholamine (e.g. norepinephrine or epinephrine), e.g. its level in the spleen, may increase, for example, by: ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, ≤ 95%, ≤ 100%, ≤ 150% or ≤ 200%.

For example, the inventors found that stimulating a splenic arterial nerve can decrease the level of a pro-inflammatory cytokine (e.g. TNFα) in the serum by 30%-60%.

Pro-inflammatory cytokines are known in the art. Examples of these include tumor necrosis factor (TNF; also known as TNFα or cachectin), interleukin (IL)-1α, IL-1β, IL-2; IL-5, IL-6, IL-8, IL-15, IL 18, interferon γ (IFN-γ); platelet-activating factor (PAF), thromboxane; soluble adhesion molecules; vasoactive neuropeptides; phospholipase A2; plasminogen activator inhibitor (PAI-1); free radical generation; neopterin; CD14; prostacyclin; neutrophil elastase; protein kinase; monocyte chemotactic proteins 1 and 2 (MCP-1, MCP-2); macrophage migration inhibitory factor (MIF), high mobility group box protein 1 (HMGB-1), and other known factors.

Anti-inflammatory cytokines are also known in the art. Examples of these include IL-4, IL-10, IL-17, IL-13, IL-1α, and TNFα receptor.

It will be recognized that some of the pro-inflammatory cytokines may act as anti-inflammatory cytokines in certain circumstances, and vice-versa. Such cytokines are typically referred to as pleiotropic cytokines.

In some embodiments, stimulation of the splenic arterial nerve may result in: (a) a decrease in the level of an anti-inflammatory cytokine in the plasma or serum by ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, or ≤ 95%; and/or (b) an increase in the level of a pro-inflammatory cytokine in the plasma or serum by ≤ 5%, ≤ 10%, ≤ 15%, ≤ 20%, ≤ 25%, ≤ 30%, ≤ 35%, ≤ 40%, ≤ 45%, ≤ 50%, ≤ 60%, ≤ 70%, ≤ 80%, ≤ 90%, ≤ 95%, ≤ 100%, ≤ 150% or ≤ 200%.

In this context the invention may be useful for increasing an immune response in a subject. For example, increasing an immune response or a pro-inflammatory response may be beneficial in a subject who is immunocompromised and thus in need of increasing pro-inflammatory cytokines for inducing beneficial pro-inflammatory responses. This may be particularly beneficial in immunocompromised subjects who are particularly vulnerable to infections. Examples of immunocompromised subjects in which this embodiment of the invention may be useful include, but are not limited to, subjects undergoing chemotherapy, subjects with HIV or AIDS, subjects taking a course of steroids, and subjects with immunosenescence, for example, subjects with age-associated immunodeficiency. In some embodiments, the invention may be used to increase a pro-inflammatory response in a subject wherein that subject is undergoing or is about to undergo a therapy in which immunocompromisation is an undesired side effect of that therapy. In other embodiments, the invention is useful for inducing a pro-inflammatory response to boost the acquisition of resistance provided by a vaccine. In other words, the neurostimulation device of the invention may be used in a method of vaccination, e.g. to boost the efficacy of a vaccine.

Factors involved in immune responses may be useful measurable parameters in the context of the invention, for example, TGF, PDGF, VEGF, EGF, FGF, I-CAM, nitric oxide.

Chemokines may also be useful measurable parameters in the context of the invention, such as 6cKine and MIP3beta, and chemokine receptors, including CCR7 receptor.

Changes in immune cell population (Langerhans cells, dendritic cells, lymphocytes, monocytes, macrophages), or immune cell surface co-stimulatory molecules (Major Histocompatibility, CD80, CD86, CD28, CD40) may also be useful measurable parameters in the context of the invention. Applying a signal to the nerves according to the invention can cause a reduction in the total counts of circulating or tissue-specific (e.g. joint-specific in the case of rheumatoid arthritis) leukocytes (including monocytes and macrophages, lymphocytes, neutrophils, etc.).

Factors involved in the inflammatory cascade may also be useful measurable parameters in the context of the invention. For example, the signal transduction cascades include factors such as NFκ-B, Egr-1, Smads, toll-like receptors, and MAP kinases.

Methods of assessing these physiological parameters are known in the art. Detection of any of the measurable parameters may be done before, during and/or after modulation of neural activity in the nerve.

For example, a cytokine, chemokine, or a catecholamine (e.g. norepinephrine or epinephrine) may be directly detected, e.g. by ELISA. Alternatively, the presence or amount of a nucleic acid, such as a polyribonucleotide, encoding a polypeptide described herein may serve as a measure of the presence or amount of the polypeptide. Thus, it will be understood that detecting the presence or amount of a polypeptide will include detecting the presence or amount of a polynucleotide encoding the polypeptide.

Quantitative changes of the biological molecules (e.g. cytokines) can be measured in a living body sample such as urine or plasma. Detection of the biological molecules may be performed directly on a sample taken from a subject, or the sample may be treated between being taken from a subject and being analyzed. For example, a blood sample may be treated by adding anti-coagulants (e.g. EDTA), followed by removing cells and cellular debris, leaving plasma containing the relevant molecules (e.g. cytokines) for analysis. Alternatively, a blood sample may be allowed to coagulate, followed by removing cells and various clotting factors, leaving serum containing the relevant molecules (e.g. cytokines) for analysis.

In the embodiments where the signal is applied whilst the subject is asleep, the invention may involve determining the subject's circadian rhythm phase markers, such as the level of cortisol (or its metabolites thereof), the level of melatonin (or its metabolites thereof) or core body temperature. Cortisol or melatonin levels can be measured in the blood (e.g. plasma or serum), saliva or urine. Methods of determining the levels of these markers are known in the art, e.g. by enzyme-linked immunosorbent assay (ELISA) or radioimmunoassay. If measurements of the subject's circadian rhythm phase markers indicate circadian oscillations of inflammatory markers which may beneficially be regulated by application of a signal with a neurostimulation device or system of the invention, then application of the signal at night at a suitable periodicity according to the subject's circadian rhythm may be appropriate.

As used herein, a physiological parameter is not affected by the modulation (e.g. stimulation) of the splenic neural activity if the parameter does not change (in response to nerve modulation) from the normal value or normal range for that value of that parameter exhibited by the subject or subject when no intervention has been performed, i.e. it does not depart from the baseline value for that parameter. Such a physiological parameter may be arterial pressure, heart rate or glucose metabolism. Suitable methods for determining changes in any these physiological parameters would be appreciated by the skilled person.

The skilled person will appreciate that the baseline for any neural activity in a subject need not be a fixed or specific value, but rather can fluctuate within a normal range or may be an average value with associated error and confidence intervals. Suitable methods for determining baseline values are well known to the skilled person.

As described herein, a physiological parameter is determined in a subject when the value for that parameter exhibited by the subject at the time of detection is determined. A detector (e.g. a physiological sensor subsystem, a physiological data processing module, a physiological sensor, etc.) is any element able to make such a determination.

Thus, in certain embodiments, the method according to this aspect of the invention further comprises a step of determining one or more physiological parameters of the subject, wherein the signal is applied only when the determined physiological parameter meets or exceeds a predefined threshold value. In such embodiments wherein more than one physiological parameter of the subject is determined, the signal may be applied when any one of the determined physiological parameters meets or exceeds its threshold value, alternatively only when all of the determined physiological parameters meet or exceed their threshold values. In certain embodiments, the signal is applied by a system of the invention, which in addition to the neurostimulation device comprises at least one detector configured to determine the one or more physiological parameters of the subject.

In certain embodiments, the physiological parameter is an action potential or pattern of action potentials in a nerve of the subject, wherein the action potential or pattern of action potentials is associated with the disease, disorder or condition to be treated.

A predefined threshold value for a physiological parameter is defined elsewhere herein.

A subject of the invention may, in addition to being treated with a neurostimulation device or system according to the invention, receive medicine for their disease, disorder or condition, as discussed elsewhere herein. In the methods of the invention, anticoagulant therapy, e.g. with heparin, may be administered to the subject prior to, following, and/or simultaneously with the application of the neurostimulation device of the invention.

### Suitable forms of an electrical signal

The neurostimulation device according to the invention applies an electrical signal via at least one electrode which is placed in proximity to, i.e. in a signalling relationship with, a splenic arterial nerve when the distal end of the catheter or stent of the neurostimulation device is inserted into a blood vessel, for example a splenic artery. The electrode may be said to be placed in signalling contact with the splenic arterial nerve. As used herein, "signalling contact" is where at least part of the electrical signal applied via the at least one electrode is received at the nerve.

Electrical signals applied according to the invention (in any medical setting described herein) may be non-destructive. As used herein, a "non-destructive signal" is a signal that, when applied, does not irreversibly damage the underlying neural signal conduction ability of the nerve. That is, application of a non-destructive signal maintains the ability of the nerve or fibers thereof, or other nerve tissue to which the signal is applied, to conduct action potentials when application of the signal ceases, even if that conduction is in practice artificially stimulated as a result of application of the non-destructive signal.

Electrical signals applied according to the invention may be a voltage or a current waveform (e.g. constant voltage or a constant current waveform).

The electrical signal may be characterized by one or more electrical signal parameters. The electrical signal parameters include waveform, frequency, and amplitude.

Alternatively or additionally, the electrical signal may be characterized by the pattern of application of the electrical signal to the nerve. The pattern of application refers to the timing of the application of the electrical signal to the nerve. The pattern of application may be continuous application or periodic application.

Continuous application refers to a situation in which the electrical signal is applied to the nerve in a continuous manner. In embodiments where the electrical signal is a series of pulses, the gaps between those pulses (i.e. between the pulse width and the phase duration) do not mean that the signal is not continuously applied.

Periodic application refers to where the electrical signal is applied to the nerve in a repeating pattern (e.g. an on-off pattern).

In the context of the treatment of a disease, disorder or condition associated with inflammation, e.g. an inflammatory disorder, e.g. an autoimmune disorder, the pattern of application of the electrical signal may be continuous application, periodic application and/or episodic application. Episodic application refers to where the electrical signal is applied to the nerve for a discrete number of episodes throughout a day. Each episode may be defined by a set duration or a set number of iterations of the electrical signal. Where the electrical signal is applied periodically and episodically, it means that the signal is applied in a periodic manner for each episode of application. Where the electrical signal is applied continuously and episodically, it means that the signal is applied in a continuous manner for each episode of application.

The inventors have found preferred electrical signal parameters and patterns of signal application for stimulating neural activity in a splenic arterial nerve by applying the signal to the application site for use in accordance with the invention, which parameters and/or patterns lead to increased immunosuppressive effects while reducing possible systemic effects when stimulating neural activity in said nerve. The preferred signal parameters and patterns of application are discussed in detail below.

The inventors have also found improved waveforms of the electrical signal which decrease the pulse height required in order to stimulate neural activity in a human nerve supplying the spleen, whilst reducing the burden on the stimulator. The improved waveforms are discussed in detail below.

### Waveform

Modulation (e.g. stimulation) of a nerve e.g. supplying the spleen can be achieved using electrical signals applied by the neurostimulation device (stent) of the invention.

A pulse train comprises a plurality of sequential pulses, where each pulse may be characterized by pulse width, pulse height and/or interphase delay. Pulse width refers to a width (or time duration) of a primary phase of the waveform. In some cases where a pulse comprises a first phase that is the primary phase and a second phase which is the recovery phase, for example an anodic and/or a cathodic phase, the pulse width refers to a width (or duration) of the first phase. A pulse duration refers to the time duration during which the pulse is applied or delivered for. This may also be referred to as a stimulation time.

Interphase delay refers to the time period from the end of a pulse to the start of the next pulse. Pulse height, which is also referred to as pulse amplitude, refers to the amplitude of current of the pulse, typically measured in amps.

Pulse width and pulse height are preferably constant for all of the pulses in the pulse train. Likewise, interphase delay is preferably constant between all of the pulses in the pulse train.

Through experimental studies, the inventors have found improved waveforms of the electrical signal which decrease the pulse height required in order to stimulate neural activity in a human nerve supplying the spleen, thereby optimizing the biological efficacy and reproducibility of stimulation parameters of the electrical signal for use in humans whilst reducing the burden on the stimulator. Thus, the electrical signal may comprise a pulse train having a pulse width > 0.1 ms, optionally ≥ 0.4ms, optionally ≥ 1ms, optionally > 1ms.

Additionally or alternatively, the pulse width may be ≤ 5 ms, optionally ≤ 3 ms, optionally ≤ 2 ms. Optionally, the pulse width may be between 0.1 and 5 ms, optionally between 0.4 and 4 ms, optionally between 1 and 3ms, optionally between 1.5 and 2.5 ms, optionally between 1.75 ms and 2.25 ms, optionally between 1.9 ms and 2.1 ms, optionally 2 ms. Moreover, the pulse train may have an interphase delay of ≤ 0.3 ms, more optionally ≤ 0.25 ms. Additionally or alternatively, the interphase delay may be ≥ 0ms, ≥ 0.1 ms, optionally ≥ 0.2 ms, more optionally 0.2 ms.

With an increased pulse width, a decrease in the pulse height required to stimulate neural activity in a human splenic nerve is observed. The pulse height required to stimulate neural activity in a nerve is also referred to herein as the 'stimulation threshold' and the 'pulse height threshold'.

The inclusion of an interphase delay may reduce the threshold of pulse height required to stimulate neural activity in a human splenic nerve. Therefore, in some examples, the pulse train may have an interphase delay.

Longer interphase delays may produce greater reductions in pulse height threshold. Accordingly, the interphase delay may have a lower limit of ≥ 0 ms, ≥0.1ms,optionally ≥ 0.15 ms, optionally ≥ 0.19 ms, optionally ≥ 0.2 ms. At interphase delays greater than 0.3 ms it was found that there is no further reduction in pulse height threshold. Accordingly, the upper limit of interphase delay of the pulse train may be ≤ 0.3 ms, more optionally ≤ 0.25 ms. Any combination of the upper and lower limits of interphase delay is possible. Preferred ranges of interphase delay include between 0.1 ms and 0.3 ms, and between 0.2 ms and 0.25 ms. The pulses are optionally square pulses. However, other pulse waveforms such as sawtooth, sinusoidal, triangular, trapezoidal, quasitrapezodial or complex waveforms may also be used with the invention.

The pulses may be biphasic in nature. The term "biphasic" refers to a pulse which applies to the nerve over time both a positive and negative charge (anodic and cathodic phases). For biphasic pulses, the pulse width includes the time duration of a primary phase of the waveform, for example the anodic phase or the cathodic phase. The primary phase may also be referred to herein as the stimulation phase.

The pulses may be charge-balanced. A charge-balanced pulse refers to a pulse which, over the period of the pulse, applies equal amounts (or thereabouts) of positive and negative charge to the nerve. The biphasic pulses are preferably charge-balanced.

The pulses may be symmetric or asymmetric. A symmetric pulse is a pulse where the waveform when applying a positive charge to the nerve is symmetrical to the waveform when applying a negative charge to the nerve. An asymmetric pulse is a pulse where the waveform when applying a positive charge to the nerve is not symmetrical with the waveform when applying a negative charge to the nerve.

**If** the biphasic pulse is asymmetric, but remains charged balanced, then the areas of the opposing phases must equal. Amplitude (see below) can be reduced, but the pulse width would need to be extended to ensure the area under the curve is matched.

In an exemplary embodiment, the waveform is a pulse train with biphasic, asymmetric, charge balanced square pulses.

### Amplitude

For the purpose of the invention, the amplitude is referred to herein in terms of charge density per phase. Charge density per phase applied to the nerve by the electrical signal is defined as the integral of the current over one phase (e.g. over one phase of the biphasic pulse in the case of a charge-balanced biphasic pulse) over a stimulating electrode surface area. Thus, charge density per phase applied to the nerve by the electrical signal is the charge per phase per unit of surface area of the at least one electrode intravascularly, and also the integral of the current density over one phase of the signal waveform. Put another way, the charge density per phase applied to the nerve by the electrical signal is the charge per phase applied to the nerve by the electrical signal divided by the surface area of the at least one electrode (generally the cathode) intravascularly.

The charge density per phase that is useful for the invention represents the amount of energy required to stimulate neural activity in a nerve supplying the spleen to increase immunosuppressive effects.

The inventors found the current that is useful to stimulate neural activity in a splenic arterial nerve to be between 1mA and 400mA, 1mA and 100mA; preferably between 5mA and 50mA, optionally between 5mA and 100mA, preferably between 10mA and 40mA, preferably between 20mA and 30mA.

The charge density per phase required to input to stimulate neural activity in a human splenic arterial nerve may be ≤ 4000 µC per cm² per phase, optionally between 20µC to 3500 µC per cm² per phase, optionally between 50µC to 3000 µC per cm² per phase, optionally between 200µC to 2000 µC per cm² per phase, optionally between 300µC to 1800 µC per cm² per phase, optionally between 400µC to 1500 µC per cm² per phase, optionally between 500µC to 1500 µC per cm² per phase.

For example, the charge density per phase applied by the electrical signal may be ≤ 100 µC per cm² per phase, ≤ 150 µC per cm² per phase, ≤ 200 µC per cm² per phase, ≤ 250 µC per cm² per phase, ≤ 300 µC per cm² per phase, ≤ 400 µC per cm² per phase, ≤ 500 µC per cm² per phase, ≤ 750 µC per cm² per phase, ≤ 1000 µC per cm² per phase, ≤ 1250 µC per cm² per phase, or ≤ 1500 µC per cm² per phase. Additionally or alternatively, the charge density per phase applied by the electrical signal may be ≥ 50 µC per cm² per phase, ≥ 100 µC per cm² per phase, ≥ 150 µC per cm² per phase, ≥ 200 µC per cm² per phase, ≥ 250 µC per cm² per phase, ≥ 300 µC per cm² per phase, ≥ 400 µC per cm² per phase, ≥ 500 µC per cm² per phase, ≥ 750 µC per cm² per phase, ≥ 1000 µC per cm² per phase, or ≥ 1250 µC per cm².

The total charge applied to the nerve by the electrical signal in any given time period is a result of the charge density per phase of the signal, in addition to the frequency of the signal, the pattern of application of the signal and the surface area of at least one electrode intravascularly. The frequency of the signal, the pattern of application of the signal and the surface area of at least one electrode intravascularly are discussed further herein.

It will be appreciated by the skilled person that the amplitude of an applied electrical signal necessary to achieve the intended stimulation of the neural activity will depend upon the positioning of the electrode and the associated electrophysiological characteristics (e.g. impedance). It is within the ability of the skilled person to determine the appropriate current amplitude for achieving the intended modulation of the neural activity in a given subject.

It would be of course understood in the art that the electrical signal applied to the nerve would be within clinical safety margins (e.g. suitable for maintaining nerve signaling function, suitable for maintaining nerve integrity, and suitable for maintaining the safety of the subject).

The electrical parameters within the clinical safety margin would typically be determined by pre-clinical studies.

The table below demonstrates example electrical signal parameters for each corresponding recruitment level of human splenic nerve using computational models. These are example values only, where a different current amplitude or pulse width may be used depending on the electrode surface area or electrode configuration of a device to achieve a corresponding charge density. In the examples given below, the electrode area is assumed to be 0.067cm². A range around the example values provided may also be used.

| | ***Recruitment (10%)*** | | ***Recruitment (50%)*** | | ***Recruitment (100%)*** | |
|---|---|---|---|---|---|---|
| **Pulse width** | **Stimulation amplitude** | **Charge densities** | **Stimulation Amplitude** | **Charge densities** | **Stimulation Amplitude** | **Charge densities** |
| 200 *µs* | ~84.2 mA | ~250 µC/cm² | ~139.7 mA | ~417 µC/cm² | ~385.2 mA | ~1149 µC/cm² |
| *400 µs* | ~43 mA | ~250 µC/cm² | ~75.25 mA | ~449 µC/cm² | ~209.6 mA | ~1251 µC/cm² |
| 600 *µs* | ~ 28.67mA | ~250 µC/cm² | ~55.55 mA | ~497 µC/cm² | ~152.3 mA | ~1363 µC/cm² |
| 800 *µs* | ~21.5 mA | ~250 µC/cm² | ~44.79 mA | ~534 µC/cm² | ~130.8mA | ~1561 µC/cm² |
| 1000 *µs* | ~17.92mA | ~267.3 µC/cm² | ~39.41 mA | ~590 µC/cm² | ~112.9 mA | ~1685 µC/cm² |
| 2000 *µs* | ~16.12mA | ~481 µC/cm² | ~37.62 mA | ~1123 µC/cm² | ~107.5 mA | ~3208 µC/cm² |

### Periodic application

Periodic application refers to where the electrical signal is applied to the nerve in a repeating pattern. The preferred repeating pattern is an on-off pattern, where the signal is applied in a sequence of pulse trains for a first duration, referred to herein as an 'on' duration, then stopped for a second duration, referred to herein as an 'off' duration, then applied again for the first duration, then stopped again for the second duration, etc.

The periodic on-off pattern may have an on duration of between 0.1 and 10 s and an off duration of between 0.5 and 30 s. For example, the on duration may be ≤ 0.2 s, ≤ 0.5 s, ≤ 1 s, ≤ 2 s, ≤ 5 s, or ≤ 10 s. Alternatively or additionally, the on duration may be ≥ 0.1 s, ≥ 0.2 s, ≥ 0.5 s, ≥ 1 s, ≥ 2 s, or ≥ 5 s. Any combination of the upper and lower limits above for the on duration is also possible. For example, the off duration may be ≤ 1 s, ≤ 3 s, ≤ 5 s, ≤ 10 s, ≤ 15 s, ≤ 20 s, ≤ 25 s, or ≤ 30 s. Alternatively or additionally, the off duration may be ≥ 0.5 s , ≥ 1 s, ≥ 2 s, ≥ 5 s, ≥ 10 s, ≥ 15 s, ≥ 20 s, or ≤ 25 s. Any combination of the upper and lower limits above for the off duration is also possible.

In an exemplary embodiment, the periodic on-off pattern has an on duration of 0.5 s on, and an off duration of 4.5 sec off.

Periodic application may also be referred to as a duty cycled application. A duty cycle represents the percentage of time that the signal is applied to the nerve for a cycle of the periodic pattern. For example, a duty cycle of 20% may represent a periodic pattern having an on duration of 2 s, and an off duration of 10 s. Alternatively, a duty cycle of 20% may represent a periodic pattern having a on duration of 1 s, and an off duration of 5 s.

Duty cycles suitable for the present invention are between 0.1% and 100%. For example, the duty cycle may be 10%.

### Episodic application

Episodic application refers to where the electrical signal is applied to the nerve for a discrete number of episodes throughout a day. The electrical signal according to the invention may be applied for up to a maximum of twenty six episodes per day. For example, the number of episodes of signal application per day may be one, two, three, four, five, six, seven, eight, nine, ten, eleven, twelve or another number up to twenty six.

The electrical signal may be applied episodically every 2 to 3 hours. For example, the electrical signal may be applied episodically once every 2 hours, 2 hour 15 min, 2 hour 30 min, 2 hour 45 min, or 3 hours.

Each episode may be defined by a set duration or a set number of iterations of the electrical signal. In some embodiments, each episode comprises applying to the nerve between 10 and 2400 pulses of the electrical signal, optionally between 100 and 2400 pulses of the electrical signal, further optionally between 50 and 2400 pulses, e.g. between 200 and 1200 pulses of the electrical signal, between 400 and 600 pulses of the electrical signal, etc. For example, each episode may comprise applying ≤ 10, ≤ 50, ≤ 60, ≤ 100, ≤ 400, ≤ 600, ≤ 800, ≤ 1200, ≤ 1600, ≤ 2000, or ≤ 2400 pulses of the electrical signal. In another example, each episode may comprise applying ≤ 200, ≤ 400, ≤ 600, ≤ 800, ≤ 1000, or ≤ 1200 pulses of the electrical signal. In a further example, each episode may comprise applying ≤ 400, ≤ 425, ≤ 450, ≤ 475, ≤ 500, ≤ 525, ≤ 550, ≤ 575, or ≤ 600 pulses of the electrical signal.

In other embodiments, each episode comprises between 20 and 40 iterations of the periodic pattern. For example, each episode comprises applying 20, 25, 30, 35, or 40 iterations of the periodic pattern, or any number therebetween. The higher the frequency, the lower the number of iterations.

As mentioned previously, in some embodiments, the episodes may be based on the subject's sleep-wake cycle, in particular the episodes may be whilst the subject is asleep. In some such embodiments, the episodes may be applied between 10 pm and 6 am. This may also be incluenced by the surgery times. The sleep-wake cycle may be measured via known methods by detecting the subject's circadian rhythm phase markers (e.g. cortisol level, melatonin level or core body temperature), and/or a detector for detecting the subject's movements.

### Frequency

Frequency is defined as the reciprocal of the phase duration of the electrical waveform (i.e. 1/phase), or put another way the interpulse timing (pulse to pulse timing).

The inventors have found preferred frequencies for stimulating a splenic arterial nerve when using the device of the invention. In particular, the inventors have found preferred frequencies for embodiments where the electrical signal is applied periodically and for embodiments where the electrical signal is applied continuously.

In embodiments where the electrical signal is applied periodically, the electrical signal has a frequency of ≤ 300 Hz, preferably ≤ 50 Hz, more preferably ≤ 10 Hz. For example, the frequency of the electrical signal may be ≤ 50 Hz, ≤ 100 Hz, ≤ 150 Hz, ≤ 200 Hz, ≤ 250 Hz or ≤ 300 Hz. In other examples, the frequency of the electrical signal may be ≤ 10 Hz, ≤ 15 Hz, ≤ 20 Hz, ≤ 25 Hz, ≤ 30 Hz, ≤ 35 Hz, ≤ 40 Hz, ≤ 45 Hz, or ≤ 50 Hz. In further examples, the frequency may be ≤ 1 Hz, ≤ 2 Hz, ≤ 5 Hz, or ≤ 10 Hz. Additionally or alternatively, the frequency of the electrical signal may be ≥ 10 Hz, ≥ 15 Hz, ≥ 20 Hz, ≥ 25 Hz, ≥ 30 Hz, ≥ 35 Hz ≥ 40 Hz, ≥ 45 Hz, or ≥ 50 Hz. In other examples, the frequency of the electrical signal may be ≥ 0.1 Hz, ≥ 0.2 Hz, ≥ 0.5 Hz, ≥ 1 Hz, ≥ 2 Hz, or ≥ 5 Hz. Any combination of the upper and lower limits above is also possible.

In embodiments where the electrical signal is applied continuously, the electrical signal has a frequency of ≤ 50 Hz, preferably ≤ 10 Hz, more preferably ≤ 2 Hz, even more preferably ≤ 1 Hz. For example, the frequency may be ≤ 1 Hz, ≤ 2 Hz, ≤ 5 Hz, or ≤ 10 Hz. In other examples the frequency may be ≤ 0.1 Hz, ≤ 0.2 Hz, ≤ 0.3 Hz, ≤ 0.4 Hz ≤ 0.5 Hz, ≤ 0.6 Hz ≤ 0.7 Hz, ≤ 0.8 Hz, or ≤ 0.9 Hz. Additionally or alternatively, the frequency of the electrical signal may be ≥ 0.1 Hz, ≥ 0.2 Hz, ≥ 0.5 Hz, ≥ 1 Hz, ≥ 2 Hz, or ≥ 5 Hz. Any combination of the upper and lower limits above is also possible.

Where the signal waveform comprises a pulse train, the pulses are applied to the nerve at intervals according to the above-mentioned frequencies. For example, a frequency of 50 Hz results in 50 pulses being applied to the nerve per second.

### Study 1: Porcine ex-vivo comparator studies

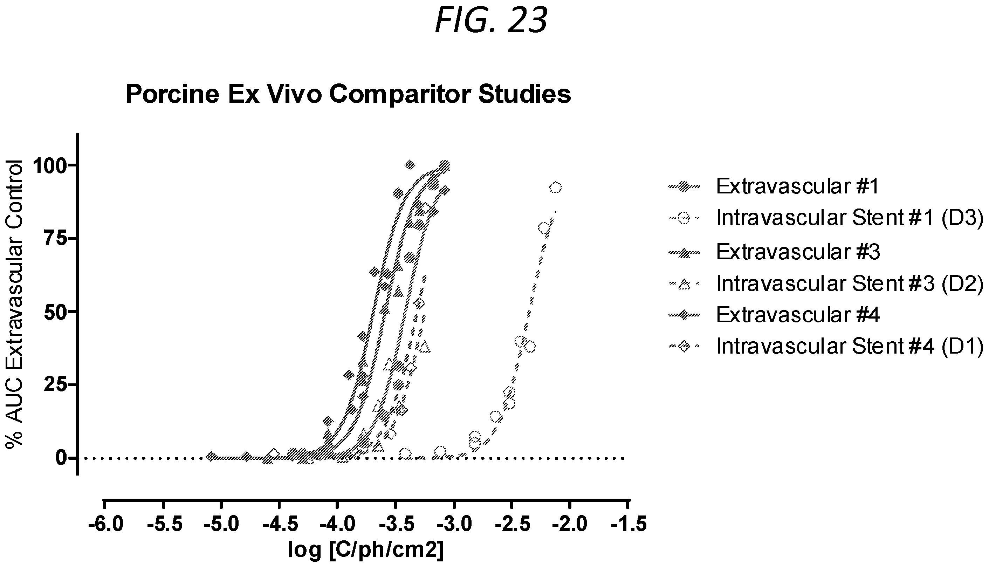

Figure 23 shows results of a study in which an ex-vivo porcine splenic artery was utilized to compare nerve activation via extravascular (filled) vs intravascular (open) interfaces. Similar symbols/colors represent paired measurements. The results of three varied stent designs are shown. Current response curves have been converted to charge density (which may also be referred to as charge density per phase) to illustrate the shift in charge density requirements when comparing extravascular to intravascular interfaces. As can be seen, x-axis refers to log[C/ph/cm²]. Thus, the scale on the x-axis which spans from -6.0 to -1.5 refers to 10^{-6.0} to 10^{-1.5}C/ph/cm². The y-axis refers to area under the curve (AUC) for eCAP measurements (response curve from each experiment has been normalised). Intravascular stimulation requirements show a dextral displacement of the charge density curve by an average factor of 5.45x, ranging from 2.18 to 11.8x. Extravascular stimulation was delivered with 0.5-2msec biphasic pulses up to 50mA. Intravascular stimulation was delivered with 1-4msec biphasic pulses up to 50mA. Internal circumferential coverage of the intravascular electrodes ranged from 100% (D1/D2) to 50% (D3) of the arterial wall.

### General Definitions

The term "comprising" encompasses "including" as well as "consisting" e.g. a composition "comprising" X may consist exclusively of X or may include something additional e.g. X + Y. The term "around" or "about" in relation to a numerical value is optional and means, for example, x+10%. Unless otherwise indicated each embodiment as described herein may be combined with another embodiment as described herein.

Any range or device value given herein may be extended or altered without losing the effect sought, as will be apparent to the skilled person.

It will be understood that the benefits and advantages described above may relate to one embodiment or may relate to several embodiments. The embodiments are not limited to those that solve any or all of the stated problems or those that have any or all of the stated benefits and advantages.

Any reference to 'an' item refers to one or more of those items. The term 'comprising' is used herein to mean including the method blocks or elements identified, but that such blocks or elements do not comprise an exclusive list and a method or apparatus may contain additional blocks or elements.

The steps of the methods described herein may be carried out in any suitable order, or simultaneously where appropriate. Additionally, individual blocks may be deleted from any of the methods without departing from the spirit and scope of the subject matter described herein. Aspects of any of the examples described above may be combined with aspects of any of the other examples described to form further examples without losing the effect sought.

It will be understood that the above description of a preferred embodiment is given by way of example only and that various modifications may be made by those skilled in the art. Although various embodiments have been described above with a certain degree of particularity, or with reference to one or more individual embodiments, those skilled in the art could make numerous alterations to the disclosed embodiments without departing from the scope of this invention.

### Embodiments

1. A stent for intravascular stimulation, the stent comprising:
   a scaffold comprising first and second scaffold structures, each scaffold structure comprising at least one substantially annular portion;
   one or more anodal electrodes formed from or electrically coupled to at least a substantially annular portion of the first scaffold structure and one or more cathodal electrodes electrically formed from or coupled to at least a substantially annular portion of the second scaffold structure;
   an anodal lead electrically coupled to the first scaffold structure to form a conductive path from the one or more anodal electrodes to a generator and a cathodal lead electrically coupled to the second scaffold structure to form a conductive path from the one or more cathodal electrodes to the generator;
   a sleeve of insulating material, wherein the scaffold structures are attached to or formed on the sleeve of insulating material and are separated from each other by a distance such that the first and second scaffold structures are electrically insulated from each other.
2. The stent of embodiment 1, wherein the one or more anodal electrodes are formed from the entirety of the substantially annular portion of the first scaffold structure and the one or more cathodal electrodes are formed from the entirety of the substantially annular portion of the second scaffold structure.
3. The stent of embodiment 1, wherein the one or more anodal electrodes are crimped or welded to the first scaffold structure and the one or more cathodal electrodes are crimped or welded to the second scaffold structure.
4. The stent of embodiment 1 or embodiment 2, wherein the anodal lead is crimped or welded to the first scaffold structure and the cathodal lead is crimped or welded to the second scaffold structure.
5. A stent for intravascular stimulation, the stent comprising:
   a scaffold formed from an electrically insulating material and comprising at least a first substantially annular portion and a second substantially annular portion spaced apart from the first by a distance;
   one or more anodal electrodes attached to the first annular portion of the scaffold and one or more cathodal electrodes attached to the second annular portion of the scaffold;
   wherein each anodal electrode is electrically coupled to an anodal lead to form a conductive path from the respective anodal electrode to a generator and each cathodal electrode is electrically coupled to a cathodal lead to form a conductive path from the respective cathodal electrode to the generator;
   a sleeve of insulating material, wherein the first and second substantially annular portions are attached to or formed on the sleeve of insulating material.
6. The stent of embodiment 5, wherein the one or more anodal electrodes are crimped to the first annular portion and the one or more cathodal electrodes are crimped to the second annular portion.
7. The stent of embodiment 5 or embodiment 6, wherein each anodal lead is crimped to its respective anodal electrode, and each cathodal lead is crimped to its respective cathodal electrode.
8. The stent of any of embodiments 5 to 7, wherein the scaffold is formed from a biocompatible stable polymer.
9. A stent for intravascular stimulation, the stent comprising:
   a scaffold formed from a material and comprising at least a first substantially annular portion and a second substantially annular portion spaced apart from the first by a distance;
   a pulse generator configured to generate electrical signals for delivery to a nerve for intravascular stimulation;
   one or more anodal electrodes formed from or attached to either the generator or the first substantially annular portion of the scaffold and one or more cathodal electrodes formed from or attached to either the generator or the second substantially annular portion of the scaffold, wherein each anodal electrode and each cathodal electrode is electrically coupled to the pulse generator;
   optionally a sleeve of insulating material, wherein the first and second substantially annular portions are attached to or formed on the sleeve of insulating material; and
   a transducer coupled to the pulse generator and configured to receive energy for delivery of power and/or communications to the pulse generator.
10. The stent of embodiment 9, wherein the scaffold is formed from an electrically insulating material, each anodal electrode is electrically coupled to the pulse generator via a respective anodal lead and each cathodal electrode is electrically coupled to the pulse generator via a respective cathodal lead.
11. The stent of embodiment 9, wherein the scaffold comprises first and second scaffold structures, the first scaffold structure comprising the first substantially annular portion and the second scaffold structure comprising the second substantially annular portion, wherein each anodal electrode is electrically coupled to the pulse generator via the first scaffold structure, and each cathodal electrode is electrically coupled to the pulse generator via the second scaffold structure, wherein the scaffold structures are attached to or formed on the sleeve of insulating material and are separated from each other by a distance such that the first and second scaffold structures are electrically insulated from each other.
12. The stent of embodiment 11, wherein the pulse generator is attached to each of the first and second scaffold structures.
13. The stent of any one of embodiments 9 to 12, wherein the RF antenna is formed from a conductor attached to, optionally weaved through, the scaffold.
14. The stent of any one of embodiments 9 to 12, wherein the RF antenna is attached to the pulse generator.
15. The stent of embodiment 11, comprising a first RF antenna formed from a conductor attached to, optionally weaved through, the first scaffold structure, and a second RF antenna formed from a conductor attached to, optionally weaved through, the second scaffold structure.
16. The stent of any preceding embodiment, wherein the at least one substantially annular portion comprises one or more hooks or projections, each hook or projection being connected at one end to the substantially annular portion and being unconnected at an opposing end to enable attachment of an electrode to the hook or projection.
17. The stent structure of embodiment 16, wherein the hooks or projections extend from the substantially annular portion in the axial direction.
18. The stent structure of embodiment 16 or embodiment 17, wherein the scaffold comprises at least two substantially annular portions, including a first annular portion and a second annular portion, spaced apart from each other along the axial direction by a distance.
19. The stent structure of embodiment 18, further comprising one or more anodal electrodes attached to the first annular portion of the scaffold and one or more cathodal electrodes attached to the second annular portion of the scaffold.
20. The stent structure of any one of embodiments 16 to 19, wherein the at least one substantially annular portion has a non-linear profile in the axial direction, optionally a meandering profile, such as a repeating chevron or zigzag profile, or a repeating sinuous or sinusoidal profile, and wherein the proximal-most points of each substantially annular portion are connected to the distal-most points of a proximally adjacent annular portion, and wherein each hook or projection extends from a distal-most point of a substantially annular portion in a proximal direction.
21. The stent structure of any one of embodiments 16 to 20, further comprising a mesh formed of a plurality of interconnected substantially annular portions, the mesh comprising apertures between adjacent substantially annular portions, and wherein each hook or projection extends from a distal-most point of a substantially annular portion in a proximal direction.
22. The stent structure of any preceding embodiment, wherein the pulse generator is configured to deliver a signal for intravascular stimulation via the anodal and cathodal electrodes for duration of between 60 seconds and 300 seconds, wherein the signal is formed of a train of pulses, and wherein:
   the pulses have a current amplitudes of between 10mA and 50mA, optionally between 20mA and 40mA; and
   the pulses have a pulse width of between 1ms to 4ms, optionally between 2ms and 3ms; and wherein either:
      A: the pulses have a frequency of between 5Hz and 15Hz, optionally between 8Hz and 12Hz, optionally 10Hz and the pulse train is delivered according to an ON/OFF cycle having a duty cycle of between 10% and 30%, optionally between 15% and 25%, more optionally 20%; or
      B: the pulses have a frequency of between 0.5Hz and 1.5Hz, optionally 1Hz and the pulse train is delivered continuously.
23. The stent of embodiment 22, wherein the pulse train is delivered for a period of between 0.2 and 0.8 seconds ON, optionally between 0.4 and 0.6 second ON, more optionally 0.5 seconds ON, followed by a period of between 2 and 7 second OFF, optionally between 3 and 6 seconds OFF, optionally between 4 and 5 seconds OFF, further optionally 4.5 seconds OFF.
24. The stent of any preceding embodiment, wherein the anodal and cathodal electrodes comprise platinum, optionally formed from an alloy of platinum and iridium, optionally in a ratio of 9:1 by weight.
25. The stent of any preceding embodiment, wherein the surfaces of the anodal and cathodal electrodes are coated possibly with PEDOT, TiNi, IrOx, PtBlack or treated using a process of laser roughening.
26. The stent of any preceding embodiment, wherein the scaffold is made from one of stainless steel, nitinol and cobalt alloy.
27. The stent of any preceding embodiment, wherein each substantially annular portion comprises between 4 and 12 electrodes, optionally 4, 6, 8, 10 or 12 electrodes.
28. The stent of any preceding embodiment, wherein the sleeve of insulating material has a first end and a second end, wherein one of the substantially annular portions comprising the anodal and cathodal electrodes is closer to the first end than the second end.
29. The stent of embodiment 28, wherein the distance between the substantially annular portion comprising the anodal electrodes and the proximal end is one of (i) the same as, (ii) greater than or (iii) less than the distance between the substantially annular portion comprising the cathodal electrodes and the distal end.
30. The stent of any of any preceding embodiment, wherein the sleeve of insulating material is formed from a flexible material, for example nylon, polyester, Pebax, or polyurethane, optionally high durometer polyurethane.
31. A system for delivery of intravascular stimulation comprising a stent according to any one of embodiments 9 to 15, or any one of embodiments 16 to 30 when dependent on any one of embodiments 9 to 15, and a radio frequency (RF) transmitter configured to transmit RF energy which, when received by the RF antenna of the stent, delivers power and/or communications to the pulse generator of the stent.
32. The system of embodiment 31, wherein the RF transmitter is removable attachable to a patient, and optionally includes attachment means such as a strap for attaching the RF transmitted to the patient.
33. The system of embodiment 31, wherein the RF transmitted is configured for subcutaneous implantation into a patient.
34. The system of embodiment 32 or embodiment 33, wherein the RF transmitter comprises a power source, optionally a portable power source, such as a battery.
35. The system of embodiment 34, when dependent on embodiment 33, wherein the power source is rechargeable and comprises a sub-system configured to recharge the power source, optionally using a wireless charging device external to the patient.
36. The system of any one of embodiments 31 to 35, wherein the RF energy is near-field, mid-field or ultrasound.
37. The system of any one of embodiments 31 to 36, wherein the stent further comprises a capacitor for storing charge received from the delivery of RF energy to the antenna
38. The system of embodiment 37, further comprising a control system configured such that:
   during a first period of time, the RF transmitter is caused to transmit RF energy such that the RF antenna receives power at a first level of current to deliver charge to the capacitor;
   during a second period of time after the first period of time has elapsed, the stent is caused to deliver intravascular stimulation continuously using charge from the capacitor, and the RF transmitter is caused not to transmit RF energy such that the RF antenna does not receive power.
39. The system of embodiment 37, further comprising a control system configured such that:
   during a first period of time, the RF transmitter is caused to transmit RF energy such that the RF antenna receives power at a first level of current to deliver charge to the capacitor;
   during a second period of time after the first period of time has elapsed, the stent is caused to deliver intravascular stimulation in a burst pattern using charge from the capacitor, and the RF transmitter is caused to transmit RF energy such that the RF antenna receives power at a second level of current, greater than the first.
40. A stent structure comprising a scaffold having at least one substantially annular portion comprising one or more hooks or projections, each hook or projection being connected at one end to the substantially annular portion and being unconnected at an opposing end to enable attachment of an electrode to the hook or projection.
41. The stent structure of embodiment 40, wherein the hooks or projections extend from the substantially annular portion in the axial direction.
42. The stent structure of embodiment 40 or embodiment 41, wherein the scaffold comprises at least two substantially annular portions, including a first annular portion and a second annular portion, spaced apart from each other along the axial direction by a distance.
43. The stent structure of embodiment 42, further comprising one or more anodal electrodes attached to the first annular portion of the scaffold and one or more cathodal electrodes attached to the second annular portion of the scaffold.
44. The stent structure of any one of embodiments 40 to 43, wherein the at least one substantially annular portion has a non-linear profile in the axial direction, optionally a meandering profile, such as a repeating chevron or zigzag profile, or a repeating sinuous or sinusoidal profile, and wherein the proximal-most points of each substantially annular portion are connected to the distal-most points of a proximally adjacent annular portion, and wherein each hook or projection extends from a distal-most point of a substantially annular portion in a proximal direction.
45. The stent structure of any one of embodiments 40 to 44, further comprising a mesh formed of a plurality of interconnected substantially annular portions, the mesh comprising apertures between adjacent substantially annular portions, and wherein each hook or projection extends from a distal-most point of a substantially annular portion in a proximal direction.
46. The stent structure of any one of embodiments 40 to 45, further comprising a sleeve of insulating material, wherein the at least one substantially annular portions are attached to or formed on the sleeve of insulating material.
47. A stent for intravascular stimulation, the stent comprising:
   a scaffold formed from a material and comprising at least a first substantially annular portion and a second substantially annular portion spaced apart from the first by a distance;
   a pulse generator configured to generate electrical signals for delivery to a nerve for intravascular stimulation;
   one or more anodal electrodes formed from or attached to either the generator or the first substantially annular portion of the scaffold and one or more cathodal electrodes formed from or attached to either the generator or the second substantially annular portion of the scaffold, wherein each anodal electrode and each cathodal electrode is electrically coupled to the pulse generator;
   optionally a sleeve of insulating material, wherein the first and second substantially annular portions are attached to or formed on the sleeve of insulating material; and
   optionally wherein the pulse generator is configured to deliver a signal for intravascular stimulation via the anodal and cathodal electrodes for duration of between 60 seconds and 300 seconds, wherein the signal is formed of a train of pulses, and/or wherein:
      the pulses have a current amplitudes of between 10mA and 50mA, optionally between 20mA and 40mA; and/or
      the pulses have a pulse width of between 1ms to 4ms, optionally between 2ms and 3ms; and/or wherein either:
         A: the pulses have a frequency of between 5Hz and 15Hz, optionally between 8Hz and 12Hz, optionally 10Hz and the pulse train is delivered according to an ON/OFF cycle having a duty cycle of between 10% and 30%, optionally between 15% and 25%, further optionally 20%.; or
         B: the pulses have a frequency of between 0.5Hz and 1.5Hz, optionally 1Hz and the pulse train is delivered continuously.
48. The stent of embodiment 47, wherein the pulse train is delivered for a period of between 0.2 and 0.8 seconds ON, optionally between 0.4 and 0.6 second ON, more optionally 0.5 seconds ON, followed by a period of between 2 and 7 second OFF, optionally between 3 and 6 seconds OFF, optionally between 4 and 5 seconds OFF, further optionally 4.5 seconds OFF.

## Claims

1. A stent for intravascular stimulation, the stent comprising:
a scaffold comprising first and second scaffold structures, each scaffold structure comprising at least one substantially annular portion;
one or more anodal electrodes formed from or electrically coupled to at least a substantially annular portion of the first scaffold structure and one or more cathodal electrodes electrically formed from or coupled to at least a substantially annular portion of the second scaffold structure;
an anodal lead electrically coupled to the first scaffold structure to form a conductive path from the one or more anodal electrodes to a generator and a cathodal lead electrically coupled to the second scaffold structure to form a conductive path from the one or more cathodal electrodes to the generator;
a sleeve of insulating material, wherein the scaffold structures are attached to or formed on the sleeve of insulating material and are separated from each other by a distance such that the first and second scaffold structures are electrically insulated from each other.

2. The stent of claim 1, wherein the one or more anodal electrodes are formed from the entirety of the substantially annular portion of the first scaffold structure and the one or more cathodal electrodes are formed from the entirety of the substantially annular portion of the second scaffold structure, or
wherein the one or more anodal electrodes are crimped or welded to the first scaffold structure and the one or more cathodal electrodes are crimped or welded to the second scaffold structure.

3. A stent for intravascular stimulation, the stent comprising:
a scaffold formed from an electrically insulating material and comprising at least a first substantially annular portion and a second substantially annular portion spaced apart from the first by a distance;
one or more anodal electrodes attached to the first annular portion of the scaffold and one or more cathodal electrodes attached to the second annular portion of the scaffold;
wherein each anodal electrode is electrically coupled to an anodal lead to form a conductive path from the respective anodal electrode to a generator and each cathodal electrode is electrically coupled to a cathodal lead to form a conductive path from the respective cathodal electrode to the generator;
a sleeve of insulating material, wherein the first and second substantially annular portions are attached to or formed on the sleeve of insulating material.

4. The stent of claim 3, wherein the one or more anodal electrodes are crimped to the first annular portion and the one or more cathodal electrodes are crimped to the second annular portion, and/or
wherein each anodal lead is crimped to its respective anodal electrode, and each cathodal lead is crimped to its respective cathodal electrode, and/or wherein the scaffold is formed from a biocompatible stable polymer.

5. The stent of any preceding claim, wherein the at least one substantially annular portion comprises one or more hooks or projections, each hook or projection being connected at one end to the substantially annular portion and being unconnected at an opposing end to enable attachment of an electrode to the hook or projection, optionally:
wherein the hooks or projections extend from the substantially annular portion in the axial direction.

6. The stent structure of claim 5, wherein the at least one substantially annular portion has a non-linear profile in the axial direction, optionally a meandering profile, such as a repeating chevron or zigzag profile, or a repeating sinuous or sinusoidal profile, and wherein the proximal-most points of each substantially annular portion are connected to the distal-most points of a proximally adjacent annular portion, and wherein each hook or projection extends from a distal-most point of a substantially annular portion in a proximal direction.

7. The stent structure of any preceding claim, wherein the pulse generator is configured to deliver a signal for intravascular stimulation via the anodal and cathodal electrodes for duration of between 60 seconds and 300 seconds, wherein the signal is formed of a train of pulses, and wherein:
the pulses have a current amplitudes of between 10mA and 50mA, optionally between 20mA and 40mA; and
the pulses have a pulse width of between 1ms to 4ms, optionally between 2ms and 3ms; and wherein either:
A: the pulses have a frequency of between 5Hz and 15Hz, optionally between 8Hz and 12Hz, optionally 10Hz and the pulse train is delivered according to an ON/OFF cycle having a duty cycle of between 10% and 30%, optionally between 15% and 25%, more optionally 20%; or
B: the pulses have a frequency of between 0.5Hz and 1.5Hz, optionally 1Hz and the pulse train is delivered continuously;
optionally wherein the pulse train is delivered for a period of between 0.2 and 0.8 seconds ON, optionally between 0.4 and 0.6 second ON, more optionally 0.5 seconds ON, followed by a period of between 2 and 7 second OFF, optionally between 3 and 6 seconds OFF, optionally between 4 and 5 seconds OFF, further optionally 4.5 seconds OFF.

8. The stent of any preceding claim, wherein the anodal and cathodal electrodes comprise platinum, optionally formed from an alloy of platinum and iridium, optionally in a ratio of 9:1 by weight, and/or
wherein the surfaces of the anodal and cathodal electrodes are coated possibly with PEDOT, TiNi, IrOx, PtBlack or treated using a process of laser roughening, and/or
wherein the scaffold is made from one of stainless steel, nitinol and cobalt alloy, and/or
wherein each substantially annular portion comprises between 4 and 12 electrodes, optionally 4, 6, 8, 10 or 12 electrodes, and/or
wherein the sleeve of insulating material is formed from a flexible material, for example nylon, polyester, Pebax, or polyurethane, optionally high durometer polyurethane.

9. The stent of any preceding claim, wherein the sleeve of insulating material has a first end and a second end, wherein one of the substantially annular portions comprising the anodal and cathodal electrodes is closer to the first end than the second end, optionally:
wherein the distance between the substantially annular portion comprising the anodal electrodes and the proximal end is one of (i) the same as, (ii) greater than or (iii) less than the distance between the substantially annular portion comprising the cathodal electrodes and the distal end.

10. A stent structure comprising a scaffold having at least one substantially annular portion comprising one or more hooks or projections, each hook or projection being connected at one end to the substantially annular portion and being unconnected at an opposing end to enable attachment of an electrode to the hook or projection.

11. The stent structure of claim 10, wherein the hooks or projections extend from the substantially annular portion in the axial direction.

12. The stent structure of claim 10 or claim 11, wherein the scaffold comprises at least two substantially annular portions, including a first annular portion and a second annular portion, spaced apart from each other along the axial direction by a distance, optionally:
further comprising one or more anodal electrodes attached to the first annular portion of the scaffold and one or more cathodal electrodes attached to the second annular portion of the scaffold.

13. The stent structure of any one of claims 10 to 12, wherein the at least one substantially annular portion has a non-linear profile in the axial direction, optionally a meandering profile, such as a repeating chevron or zigzag profile, or a repeating sinuous or sinusoidal profile, and wherein the proximal-most points of each substantially annular portion are connected to the distal-most points of a proximally adjacent annular portion, and wherein each hook or projection extends from a distal-most point of a substantially annular portion in a proximal direction.

14. The stent structure of any one of claims 5 to 9 or 10 to 13, further comprising a mesh formed of a plurality of interconnected substantially annular portions, the mesh comprising apertures between adjacent substantially annular portions, and wherein each hook or projection extends from a distal-most point of a substantially annular portion in a proximal direction.

15. The stent structure of any one of claims 10 to 13, or claim 14 when dependent on any one of claims 10 to 13, further comprising a sleeve of insulating material, wherein the at least one substantially annular portions are attached to or formed on the sleeve of insulating material.
